# EUROPÄISCHE PATENTANMELDUNG

(11) **EP 2 172 466 A1**
(43) Veröffentlichungstag der Anmeldung: **07.04.2010**
(21) Anmeldenummer: 09179928.8
(22) Anmeldetag: 22.07.2005
(51) Int. Cl.: C07D 401/04, A01N 43/54

(54) **2-(Pyridin-2-yl)-pyrimidine und ihre Verwendung zur Bekämpfung von Schadpilzen**

(30) Priorität: 23.07.2004 DE 102004035736
(62) Teilanmeldung aus: 05761662.5
(71) Anmelder: BASF SE, 67056 Ludwigshafen (DE)
(72) Erfinder: GRAMMENOS, Wassilios, 67071 Ludwigshafen (DE); GROTE, Thomas, 67157 Wachenheim (DE); BLETTNER, Carsten, 67063 Ludwighafen (DE); GEWEHR, Markus, 56288 Kastellaun (DE); HÜNGER, Udo, 67245 Lambsheim (DE); MÜLLER, Bernd, 67227 Frankenthal (DE); RHEINHEIMER, Joachim, 67063 Ludwigshafen (DE); SCHÄFER, Peter, 67308 Ottersheim (DE); SCHIEWECK, Frank, 67098 Bad Dürkheim (DE); SCHWÖGLER, Anja, 67112 Mutterstadt (DE); SCHÖFL, Ulrich, US-Apex, NC 27523 (US); KÖHLE, Harald, 67273 Bobenheim (DE); STRATHMANN, Siegfried, 67117 Limburgerhof (DE); SCHERER, Maria, 76829 Landau (DE); STIERL, Reinhard, TW-83345 Kaohsiung County (TW); RETHER, Jan, 67655 Kaiserslautern (DE)
(74) Vertreter: Reitstötter - Kinzebach

(57) **Zusammenfassung**

Die vorliegende Erfindung betrifft 2-(Pyridin -2-yl)-pyrimidine Verbindungen der allgemeinen Formel I und ihre Verwendung zur Bekämpfung von Schadpilzen sowie Pflanzenschutzmittel, die derartige Verbindungen als wirksamen Bestandteil enthalten. 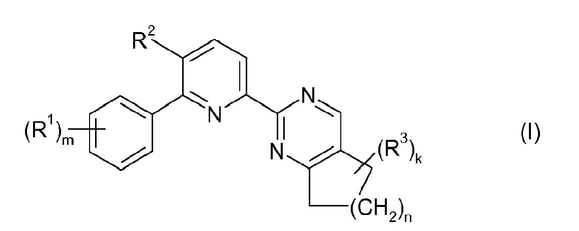 Hierin steht k für 0, 1, 2 oder 3, m für 0, 1, 2, 3, 4 oder 5, n für 1, 2, 3, 4 oder 5;
R¹ bedeuten unabhängig voneinander Halogen, OH, CN, NO₂ C₁-C₄-Alkyl, C₁-C₄-Halogenalkyl, C₁-C₄-Alkoxy, C₁-C₄-Halogenalkoxy, C₂-C₄-Alkenyl, C₂-C₄-Alkinyl, C₃-C₈-Cycloalkyl, C₁-C₄-Alkoxy-C₁-C₄-alkyl, Amino, Phenoxy, das gegebenenfalls durch Halogen oder C₁-C₄-Alkyl substituiert ist, NHR, NR₂, C(R^{a})=N-OR^{b}, S(=O)ₚA¹ oder C(=O)A², oder zwei an benachbarte C-Atome gebundene Reste R¹ können auch gemeinsam für eine Gruppe -O-Alk-O- stehen, worin Alk für lineares oder verzweigtes C₁-C₄-Alkylen steht, worin 1, 2, 3 oder 4 Wasserstoffatome auch durch Halogen ersetzt sein können;
R² C₁-C₄-Halogenalkyl, C₁-C₄-Alkoxy, C₁-C₄-Halogenalkoxy, Hydroxy, Halogen, CN oder NO₂ bedeutet; wobei R² auch Wasserstoff oder C₁-C₄-Alkyl bedeuten kann, wenn wenigstens eine der drei folgenden Bedingungen erfüllt ist:
- n steht für 3, 4 oder 5,
- k steht für 1, 2 oder 3,
- für m ≠ 0 steht wenigstens einer der Reste R¹ für einen von Halogen, C₁-C₄-Alkyl, C₁-C₄-Alkoxy und C₁-C₄-Halogenalkyl verschieden Rest; und
R³ für C₁-C₄-Alkyl.

## Beschreibung

Die vorliegende Erfindung betrifft 2-(Pyridin -2-yl)-pyrimidine und ihre Verwendung zur Bekämpfung von Schadpilzen sowie Pflanzenschutzmittel, die derartige Verbindungen als wirksamen Bestandteil enthalten.

Die EP-A 234 104 beschreibt 2-(Pyridin-2-yl)-pyrimidine, die in der 6-Position des Pyridinrestes eine Alkylgruppe aufweisen und die in der 3,4-Position des Pyrimidinrings einen anellierten gesättigten 5- oder 6-Ring aufweisen können. Die Verbindungen sind zur Bekämpfung von pflanzen pathogenen Pilzen (Schadpilzen) geeignet.

Aus der US 4,873,248 sind 2-(Pyridin-2-yl)-pyrimidine mit fungizider Wirkung bekannt, die in der 4-Position des Pyrimidinrings einen gegebenenfalls substituierten Phenylring tragen.

Die EP-A 259 139 beschreibt 2-(Pyridin-2-yl)-pyrimidine der allgemeinen Formel A 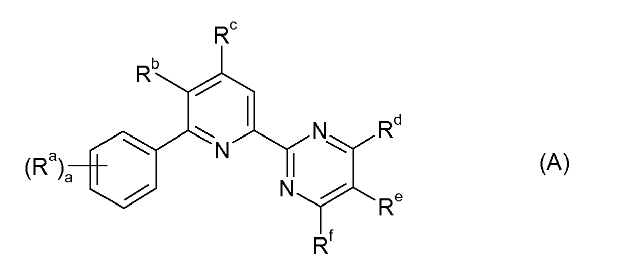 worin a für 0, 1, 2, 3, 4 oder 5 steht, R^{a} Halogen, Niederalkyl, Niederalkoxy oder Halogenalkyl bedeutet, R^{b} und R^{c} unabhängig voneinander für Wasserstoff oder C₁-C₄-Alkyl stehen, R^{d} Wasserstoff oder Niederalkyl bedeutet, R^{e} Wasserstoff, Niederalkyl oder Halogen bedeutet oder gemeinsam mit R^{d} für Propan-1,3-diyl oder Butan-1,4-diyl steht und R^{f} unter anderem Wasserstoff, Alkyl, Niederalkoxy oder Niederalkylthio bedeutet.

Die aus dem Stand der Technik bekannten 2-(Pyridin-2-yl)-pyrimidine sind hinsichtlich ihrer fungiziden Wirkung teilweise nicht zufriedenstellend oder besitzen unerwünschte Eigenschaften, wie eine geringe Nutzpflanzenverträglichkeit.

Der vorliegenden Erfindung liegt daher die Aufgabe zugrunde, neue Verbindungen mit besserer fungizider Wirksamkeit und/oder einer besseren Nutzpflanzenverträglichkeit bereitzustellen.

Diese Aufgabe wird überraschenderweise gelöst durch 2-(Pyridin-2-yl)-pyrimidinVerbindungen der allgemeinen Formel I 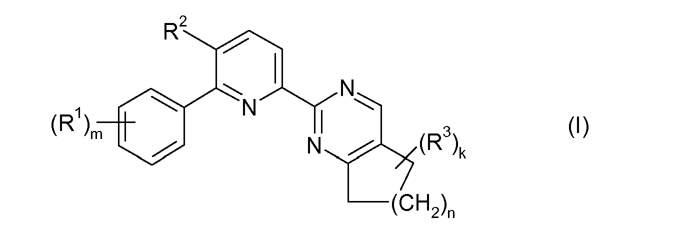 worin:
- k: für 0, 1, 2 oder 3 steht;
- m: für 0, 1, 2, 3, 4 oder 5 steht;
- n: für 1, 2, 3, 4 oder 5 steht;
- R¹: unabhängig voneinander Halogen, OH, CN, NO₂, C₁-C₄-Alkyl, C₁-C₄-Halogenalkyl, C₁-C₄-Alkoxy, C₁-C₄-Halogenalkoxy, C₂-C₄-Alkenyl, C₂-C₄-Alkinyl, C₃-C₈-Cycloalkyl, C₁-C₄-Alkoxy-C₁-C₄-alkyl, Amino, Phenoxy, das gegebenenfalls durch Halogen oder C₁-C₄-Alkyl substituiert ist, NHR, NR₂, C(R^{a})=N-OR^{b}, S(=O)ₚA¹ oder C(=O)A² bedeuten, wobei p, R, R^{a}, R^{b}, A¹ und A² die folgenden Bedeutungen haben:
R C₁-C₄-Alkyl oder C₁-C₄-Alkylcarbonyl,
R^{a} Wasserstoff oder C₁-C₄-Alkyl;
R^{b} C₁-C₄-Alkyl, C₃-C₄-Alkenyl oder C₃-C₄-Alkinyl,
p 0, 1 oder 2,
A¹ C₁-C₄-Alkyl, oder für p = 2 auch NH₂, C₁-C₄-Alkylamino oder Di-(C₁-C₄-alkyl)amino, und
A² Wasserstoff, Hydroxy, C₁-C₄-Alkyl, C₁-C₄-Alkylamino, Di-(C₁-C₄-alkyl)amino, C₂-C₄-Alkenyl, C₁-C₄-Alkoxy oder C₁-C₄-Halogenalkoxy; oder zwei an benachbarte C-Atome gebundene Reste R¹ auch gemeinsam für eine Gruppe -O-Alk-O- stehen können, worin Alk für lineares oder verzweigtes C₁-C₄-Alkylen steht, worin 1, 2, 3 oder 4 Wasserstoffatome auch durch Halogen ersetzt sein können;
- R²: C₁-C₄-Halogenalkyl, C₁-C₄-Alkoxy, C₁-C₄-Halogenalkoxy, Hydroxy, Halogen, CN oder NO₂ bedeutet; wobei R² auch Wasserstoff oder C₁-C₄-Alkyl bedeuten kann, wenn wenigstens eine der drei folgenden Bedingungen erfüllt ist:
- n steht für 3, 4 oder 5,
- k steht für 1, 2 oder 3,
- für m ≠ 0 steht wenigstens einer der Reste R¹ für einen von Halogen, C₁-C₄-Alkyl, C₁-C₄-Alkoxy und C₁-C₄-Halogenalkyl verschieden Rest; und
- R³: für C₁-C₄-Alkyl steht;
und durch die landwirtschaftlich verträglichen Salze der Verbindungen I.

Gegenstand der vorliegenden Erfindung sind somit die 2-(Pyridin-2-yl)-pyrimidine der allgemeinen Formel I und deren landwirtschaftlich verträglichen Salze.

Gegenstand der vorliegenden Erfindung ist weiterhin die Verwendung der 2-(Pyridin-2-yl)-pyrimidine der allgemeinen Formel I und ihrer landwirtschaftlich verträglichen Salze zur Bekämpfung von pflanzen pathogenen Pilzen (= Schadpilzen) sowie ein Verfahren zur Bekämpfung von pflanzenpathogenen Pilzen, das dadurch gekennzeichnet ist, dass man die Pilze, oder die vor Pilzbefall zu schützenden Materialien, Pflanzen, den Boden oder Saatgüter mit einer wirksamen Menge einer Verbindung der allgemeinen Formel I und/oder mit einem landwirtschaftlich verträglichen Salz von I behandelt.

Gegenstand der vorliegenden Erfindung ist weiterhin ein Mittel zur Bekämpfung von Schadpilzen, enthaltend wenigstens eine 2-(Pyridin-2-yl)-pyrimidin-Verbindung der allgemeinen Formel I und/oder ein landwirtschaftlich verträgliches Salz davon und wenigstens einen flüssigen oder festen Trägerstoff.

Die Verbindungen der Formel I und deren Tautomere können je nach Substitutionsmuster ein oder mehrere Chiralitätszentren aufweisen und liegen dann als reine Enantiomere oder reine Diastereomere oder als Enantiomeren- oder Diastereomerengemische vor. Gegenstand der Erfindung sind sowohl die reinen Enantiomere oder Diastereomere als auch deren Gemische.

Unter landwirtschaftlich brauchbaren Salzen kommen vor allem die Salze derjenigen Kationen oder die Säureadditionssalze derjenigen Säuren in Betracht, deren Kationen beziehungsweise Anionen die fungizide Wirkung der Verbindungen I nicht negativ beeinträchtigen. So kommen als Kationen insbesondere die Ionen der Alkalimetalle, vorzugsweise Natrium und Kalium, der Erdalkalimetalle, vorzugsweise Calcium, Magnesium und Barium, und der Übergangsmetalle, vorzugsweise Mangan, Kupfer, Zink und Eisen, sowie das Ammoniumion, das gewünschtenfalls ein bis vier C₁-C₄-Alkylsubstituenten und/oder einen Phenyl- oder Benzylsubstituenten tragen kann, vorzugsweise Diisopropylammonium, Tetramethylammonium, Tetrabutylammonium, Trimethylbenzylammonium, des weiteren Phosphoniumionen, Sulfoniumionen, vorzugsweise Tri(C₁-C₄-alkyl)sulfonium und Sulfoxoniumionen, vorzugsweise Tri(C₁-C₄-alkyl)sulfoxonium, in Betracht.

Anionen von brauchbaren Säureadditionssalzen sind in erster Linie Chlorid, Bromid, Fluorid, Hydrogensulfat, Sulfat, Dihydrogenphosphat, Hydrogenphosphat, Phosphat, Nitrat, Hydrogencarbonat, Carbonat, Hexafluorosilikat, Hexafluorophosphat, Benzoat, sowie die Anionen von C₁-C₄-Alkansäuren, vorzugsweise Formiat, Acetat, Propionat und Butyrat. Sie können durch Reaktion von I mit einer Säure des entsprechenden Anions, vorzugsweise der Chlorwasserstoffsäure, Bromwasserstoffsäure, Schwefelsäure, Phosphorsäure oder Salpetersäure, gebildet werden.

Bei den in den vorstehenden Formeln angegebenen Definitionen der Variablen werden Sammelbegriffe verwendet, die allgemein repräsentativ für die jeweiligen Substituenten stehen. Die Bedeutung Cₙ-Cₘ gibt die jeweils mögliche Anzahl von Kohlenstoffatomen in dem jeweiligen Substituenten oder Substituententeil an:
**Halogen:** Fluor, Chlor, Brom und Jod;
**Alkyl sowie alle Alkylteile in Alkoxy, Alkoxyalkyl, Alkylamino und Dialkylamino:** gesättigte, geradkettige oder verzweigte Kohlenwasserstoffreste mit 1 bis 4 Kohlenstoffatomen, z.B. C₁-C₄-Alkyl wie Methyl, Ethyl, Propyl, 1-Methylethyl, Butyl, 1-Methylpropyl, 2-Methylpropyl, 1,1-Dimethylethyl;
**Halo(gen)alkyl:** geradkettige oder verzweigte Alkylgruppen mit 1 bis 4 Kohlenstoffatomen (wie vorstehend genannt), wobei in diesen Gruppen teilweise oder vollständig die Wasserstoffatome durch Halogenatome wie vorstehend genannt und insbesondere durch Fluor oder Chlor ersetzt sein können, insbesondere C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl und 1,1,1-Trifluorprop-2-yl;
**Alkenyl:** einfach ungesättigte, geradkettige oder verzweigte Kohlenwasserstoffreste mit 2 bis 4 oder 3 bis 4 Kohlenstoffatomen und einer Doppelbindung in einer beliebigen Position, z. B. Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl;
**Alkinyl:** geradkettige oder verzweigte Kohlenwasserstoffgruppen mit 2 bis 4 oder 3 bis 4 Kohlenstoffatomen und einer Dreifachbindung in einer beliebigen Position, z. B. Ethinyl, 1-Propinyl, 2-Propinyl, 1-Butinyl, 2-Butinyl, 3-Butinyl, 1-Methyl-2-propinyl;
**Cycloalkyl:** monocyclische, gesättigte Kohlenwasserstoffgruppen mit 3 bis 8, vorzugsweise bis 6 Kohlenstoffringgliedern, wie Cyclopropyl, Cyclobutyl, Cyclopentyl und Cyclohexyl;
**Alkylamino:** für einen über eine NH-Gruppe gebundene Alkylgruppe, worin Alkyl für einen der zuvor genannten Alkylreste mit 1 bis 4 C-Atomen steht, wie Methylamino, Ethylamino, n-Propylamino, Isopropylamino, n-Butylamino und dergleichen;
**Dialkylamino:** für einen Rest der Formel N(Alkyl)₂, worin Alkyl für einen der zuvor genannten Alkylreste mit 1 bis 4 C-Atomen steht, z. B. für Dimethylamino, Diethylamino, Methylethylamino, N-Methyl-N-propylamino und dergleichen;
**C₁-C₄-Alkoxy:** für eine über ein Sauerstoff gebundene Alkylgruppe mit 1 bis 4 C-Atomen, z. B. Methoxy, Ethoxy, n-Propoxy, 1-Methylethoxy, Butoxy, 1-Methylpropoxy, 2-Methylpropoxy oder 1,1-Dimethylethoxy;
**C₁-C₄-Halogenalkoxy:** für einen C₁-C₄-Alkoxyrest wie vorstehend genannt, der partiell oder vollständig durch Fluor, Chlor, Brom und/oder Iod, vorzugsweise durch Fluor substituiert ist, also z. B. OCH₂F, OCHF₂, OCF₃, OCH₂Cl, OCHCl₂, OCCl₃, Chlorfluormethoxy, Dichlorfluormethoxy, Chlordifluormethoxy, 2-Fluorethoxy, 2-Chlorethoxy, 2-Bromethoxy, 2-lodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, OC₂F₅, 2-Fluorpropoxy, 3-Fluorpropoxy, 2,2-Difluorpropoxy, 2,3-Difluorpropoxy, 2-Chlorpropoxy, 3-Chlorpropoxy, 2,3-Dichlorpropoxy, 2-Brompropoxy, 3-Brompropoxy, 3,3,3-Trifluorpropoxy, 3,3,3-Trichlorpropoxy, OCH₂-C₂F₅, OCF₂-C₂F₅, 1-(CH₂F)-2-fluorethoxy, 1-(CH₂Cl)-2-chlorethoxy, 1-(CH₂Br)-2-bromethoxy, 4-Fluorbutoxy, 4-Chlorbutoxy, 4-Brombutoxy oder Nonafluorbutoxy;
**Alkylen:** für eine lineare gesättigte Kohlenwasserstoffkette mit 2 bis 6 und insbesondere 2 bis 4 C-Atomen wie Ethan-1,2-diyl, Propan-1,3-diyl, Butan-1,4-diyl, Pentan-1,5-diyl oder Hexan-1,6-diyl.

Eine erste Ausführungsform der Erfindung betrifft Verbindungen, worin n für 3, 4 oder 5 und insbesondere für 3 oder 4 steht, R² Wasserstoff, C₁-C₄-Alkyl, C₁-C₄-Halogenalkyl, C₁-C₄-Alkoxy, C₁-C₄-Halogenalkoxy, Hydroxy, Halogen, CN oder NO₂ bedeutet und die übrigen Variablen die zuvor genannten Bedeutungen aufweisen, insbesondere die im Folgenden als bevorzugt genannten Bedeutungen aufweisen.

Eine zweite Ausführungsform der Erfindung betrifft Verbindungen, worin n für 1 oder 2 und insbesondere für 2 steht, R² C₁-C₄-Halogenalkyl, C₁-C₄-Alkoxy, C₁-C₄-Halogenalkoxy, Hydroxy, Halogen, CN oder NO₂ bedeutet und die übrigen Variablen die zuvor genannten Bedeutungen aufweisen, insbesondere die im Folgenden als bevorzugt genannten Bedeutungen aufweisen.

Im Hinblick auf die Verwendung als Fungizide sind solche Verbindungen der Formel I bevorzugt, worin die Variablen m, n, k, R¹, R² und R³ unabhängig voneinander und insbesondere in Kombination die folgenden Bedeutungen aufweisen.
- m: 0 oder eine ganze Zahl 1, 2 oder 3;
- n: 1, 2 oder 3, speziell 2 oder 3;
- k: 0, 1 oder 2, speziell 0. Sofern k von 0 verschieden ist, können der/die Reste R³ an jeder beliebigen Position des gesättigten anellierten Rings angeordnet sein. In einer besonders bevorzugten Ausführungsform steht k für 0. In einer weiteren bevorzugten Ausführungsform steht k für 2. In dieser Ausführungsform sind die beiden Reste R³ vorzugsweise am gleichen Kohlenstoffatom angeordnet.
- R¹: Halogen, insbesondere Fluor, Chlor oder Brom, Cyano, OH, CHO, NO₂, NH₂, Methylamino, Dimethylamino, Diethylamino, C₁-C₄-Alkyl, insbesondere Methyl, Isopropyl oder tert.-Butyl, C₃-C₈-Cycloalkyl, speziell Cyclopropyl, Cyclopentyl oder Cyclohexyl, C₁-C₄-Alkoxy, speziell OCH₃, C₁-C₄-Alkylthio, speziell Methylthio oder Ethylthio, C₁-C₄-Haloalkyl, insbesondere C₁-C₂-Fluoralkyl, speziell CF₃,
C₁-C₄-Haloalkoxy, speziell OCHF₂ oder OCF₃, oder CO(A²), worin A² für C₁-C₄-Alkoxy, speziell OCH₃, oder für C₁-C₄-Alkyl, speziell Methyl, steht. Ebenfalls bevorzugt sind Verbindungen, worin einer der Reste R¹ für eine Gruppe C(R^{a})=NOR^{b} steht, worin R^{a} und R^{b} die zuvor genannten Bedeutungen aufweisen und worin R^{a} insbesondere für H oder CH₃ steht und R^{b} insbesondere für C₁-C₄-Alkyl, Propargyl oder Allyl steht.
Besonders bevorzugt ist R¹ ausgewählt unter Halogen, insbesondere F oder Cl, CN, C₁-C₄-Alkyl, insbesondere Methyl, Isopropyl oder tert.-Butyl, C₁-C₄-Halogenalkyl, insbesondere C₁-C₂-Fluoralkyl, speziell Trifluormethyl, C₁-C₄-Alkoxy, insbesondere Methoxy, Ethoxy, Isopropyloxy, C₁-C₄-Alkylthio, z. B. Methylthio, oder C₁-C₄-Halogenalkoxy, insbesondere OCF₃, und OCHF₂ oder einer der Reste steht für eine Gruppe C(R^{a})=N-OR^{b} oder eine Gruppe C(O)-A².
Ganz besonders bevorzugte Reste R¹ sind ausgewählt unter Methyl, F, Cl, Methoxy, Trifluormethyl und CN. Ebenfalls besonders bevorzugte Reste sind ausgewählt unter CH(=NOCH₃), C(CH₃)(=N-OCH₃), C(CH₃)(=N-OC₂H₅), C(O)CH₃ und CO₂CH₃.
- R²: C₁-C₄-Halogenalkyl, hierunter bevorzugt C₁-C₂-Fluoralkyl und speziell Trifluormethyl, C₁-C₄-Alkoxy, C₁-C₄-Halogenalkoxy, Halogen, CN oder NO₂ insbesondere C₁-C₄-Alkoxy, speziell Methoxy oder Ethoxy, C₁-C₄-Halogenalkoxy, insbesondere Trifluormethoxy oder Difluormethoxy, oder Halogen, speziell F, Cl oder Br.;
Bevorzugt sind auch solche Verbindungen der allgemeinen Formel I mit n = 3, 4 oder 5, worin R² für Wasserstoff oder insbesondere C₁-C₄-Alkyl steht und besonders bevorzugt Methyl oder Ethyl bedeutet.
Ebenfalls bevorzugt sind solche Verbindungen der Formel I mit k = 1, 2 oder 3 und speziell 2, worin R² für Wasserstoff oder insbesondere C₁-C₄-Alkyl steht und besonders bevorzugt Methyl oder Ethyl bedeutet.
- R³: Methyl oder Isopropyl.

Unter den Verbindungen der Formel I sind besonders solche bevorzugt, in der die durch (R¹)ₘ substituierte Phenylgruppe für die Gruppe der Formel P 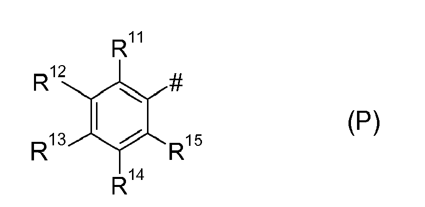 steht, worin # die Verknüpfungsstelle mit dem Pyridinring ist und R¹¹, R¹², R¹³, R¹⁴ und R¹⁵ die für R¹ angegebenen, insbesondere die als bevorzugt oder besonders bevorzugt angegebenen Bedeutungen aufweisen. In einer bevorzugten Ausführungsform ist wenigstens einer und speziell 1, 2 oder 3 der Reste R¹¹, R¹², R¹³, R¹⁴ oder R¹⁵ von Wasserstoff verschieden. In einer anderen bevorzugten Ausführungsform stehen alle Reste R¹¹, R¹², R¹³, R¹⁴ und R¹⁵ für Wasserstoff. Insbesondere bedeuten:
- R¹¹: Wasserstoff, Fluor, Chlor, CH₃, OCH₃, OCHF₂, OCF₃ oder CF₃;
- R¹², R¹⁴: unabhängig voneinander Wasserstoff, Chlor, Fluor, CH₃, OCH₃, OCHF₂, OCF₃ oder CF₃, wobei einer der Reste R¹² und R¹⁴ auch für NO₂ C(O)CH₃ oder COOCH₃ stehen kann; insbesondere stehen R¹² und R¹⁴ für Wasserstoff, Fluor, Methyl oder Trifluormethyl;
- R¹³: Wasserstoff, Fluor, Chlor, Cyano, OH, CHO, NO₂, NH₂, Methylamino, Dimethylamino, Diethylamino, C₁-C₄-Alkyl, speziell CH₃, C₂H₅, CH(CH₃)₂, C₃-C₈-Cycloalkyl, speziell Cyclopropyl, Cyclopentyl oder Cyclohexyl, C₁-C₄-Alkoxy, speziell OCH₃, C₁-C₄-Alkylthio, speziell Methylthio oder Ethylthio, C₁-C₄-Haloalkyl, speziell CF₃, C₁-C₄-Haloalkoxy, speziell OCHF₂ oder OCF₃, oder CO(A²), worin A² für C₁-C₄-Alkyl, speziell Methyl oder für C₁-C₄-Alkoxy, speziell OCH₃, steht, oder R¹² und R¹³ gemeinsam eine Gruppe O-CH₂-O bilden; und
- R¹⁵: Wasserstoff, Fluor, Chlor, oder C₁-C₄-Alkyl, speziell CH₃, insbesondere Wasserstoff oder Fluor.

Ebenfalls bevorzugt sind Verbindungen, worin R¹² oder R¹³ für eine Gruppe C(R^{a})=NOR^{b} steht, worin R^{a} und R^{b} die zuvor und insbesondere die als bevorzugt genannten Bedeutungen aufweisen.

Sofern mehr als einer der Reste R¹¹, R¹², R¹³, R¹⁴ oder R¹⁵ von Wasserstoff verschieden ist, dann ist vorteilhafterweise nur einer der von Wasserstoff verschiedenen Reste von Halogen oder Methyl verschieden. Speziell wenn einer der Reste R¹¹, R¹², R¹³, R¹⁴ oder R¹⁵ von Wasserstoff, Halogen oder Methyl verschieden ist, dann sind die verbleibenden Reste R¹¹, R¹², R¹³, R¹⁴, R¹⁵ unter Halogen und Wasserstoff ausgewählt.

Beispiele für Reste P sind insbesondere solche, worin R¹¹, R¹², R¹³, R¹⁴ und R¹⁵ gemeinsam die in einer Zeile der Tabelle A angegebenen Bedeutungen haben:

**Tabelle A:**

| | R¹¹ | R¹² | R¹³ | R¹⁴ | R¹⁵ |
|---|---|---|---|---|---|
| 1 | H | H | H | H | H |
| 2 | F | H | H | H | H |
| 3 | H | F | H | H | H |
| 4 | H | H | F | H | H |
| 5 | Cl | H | H | H | H |
| 6 | H | Cl | H | H | H |
| 7 | H | H | Cl | H | H |
| 8 | Br | H | H | H | H |
| 9 | H | Br | H | H | H |
| 10 | H | H | Br | H | H |
| 11 | F | F | H | H | H |
| 12 | F | H | F | H | H |
| 13 | F | H | H | H | F |
| 14 | H | F | F | H | H |
| 15 | H | F | H | F | H |
| 16 | Cl | Cl | H | H | H |
| 17 | Cl | H | Cl | H | H |
| 18 | Cl | H | H | Cl | H |
| 19 | H | Cl | Cl | H | H |
| 20 | H | Cl | H | Cl | H |
| 21 | F | Cl | H | H | H |
| 22 | Cl | F | H | H | H |
| 23 | F | H | Cl | H | H |
| 24 | F | H | H | Cl | H |
| 25 | F | H | H | H | Cl |
| 26 | Cl | H | F | H | H |
| 27 | H | Cl | F | H | H |
| 28 | H | F | Cl | H | H |
| 29 | H | Cl | H | F | H |
| 30 | F | H | F | H | F |
| 31 | F | F | F | F | F |
| 32 | CF₃ | H | H | H | H |
| 33 | H | CF₃ | H | H | H |
| 34 | H | H | CF₃ | H | H |
| 35 | F | Cl | H | Cl | H |
| 36 | F | H | Cl | Cl | H |
| 37 | F | H | H | Cl | Cl |
| 38 | Cl | H | H | H | CF₃ |
| 39 | Cl | H | H | CF₃ | H |
| 40 | Cl | H | CF₃ | H | H |
| 41 | OCF₃ | H | H | H | H |
| 42 | H | OCF₃ | H | H | H |
| 43 | H | H | OCF₃ | H | H |
| 44 | F | H | H | H | OCF₃ |
| 45 | F | H | H | OCF₃ | H |
| 46 | F | H | OCF₃ | H | H |
| 47 | Cl | H | H | H | OCF₃ |
| 48 | Cl | H | OCF₃ | H | H |
| 49 | OHCF₂ | H | H | H | H |
| 50 | H | OCHF₂ | H | H | H |
| 51 | H | H | OCHF₂ | H | H |
| 52 | F | H | H | H | OCHF₂ |
| 53 | F | H | OCHF₂ | H | H |
| 54 | Cl | H | H | OCHF₂ | H |
| 55 | Cl | H | OCHF₂ | H | H |
| 56 | CH₃ | H | H | H | H |
| 57 | H | CH₃ | H | H | H |
| 58 | H | H | CH₃ | H | H |
| 59 | F | H | H | H | CH₃ |
| 60 | F | H | H | CH₃ | H |
| 61 | F | H | CH₃ | H | H |
| 62 | H | F | CH₃ | H | H |
| 63 | H | CH₃ | F | H | H |
| 64 | F | CH₃ | H | H | H |
| 65 | OCH₃ | H | H | H | H |
| 66 | H | OCH₃ | H | H | H |
| 67 | F | H | H | H | OCH₃ |
| 68 | F | H | H | OCH₃ | H |
| 69 | F | H | OCH₃ | H | H |
| 70 | H | F | OCH₃ | H | H |
| 71 | Cl | H | H | H | CH₃ |
| 72 | Cl | H | CH₃ | H | H |
| 73 | H | H | C₂H₅ | H | H |
| 74 | H | H | CH(CH₃)₂ | H | H |
| 75 | H | H | C(CH₃)₃ | H | H |
| 76 | H | H | OH | H | H |
| 77 | H | H | OCH₃ | H | H |
| 78 | H | H | OC₂H₅ | H | H |
| 79 | H | H | OCH(CH₃)₂ | H | H |
| 80 | H | H | OC(CH₃)₃ | H | H |
| 81 | F | H | OCH₃ | H | H |
| 82 | F | H | OC₂H₅ | H | H |
| 83 | F | H | OCH(CH₃)₂ | H | H |
| 84 | F | H | OC(CH₃)₃ | H | H |
| 85 | H | F | OCH₃ | H | H |
| 86 | H | F | OC₂H₅ | H | H |
| 87 | H | F | OCH(CH₃)₂ | H | H |
| 88 | H | F | OC(CH₃)₃ | H | H |
| 89 | Cl | H | OCH₃ | H | H |
| 90 | Cl | H | OC₂H₅ | H | H |
| 91 | Cl | H | OCH(CH₃)₂ | H | H |
| 92 | Cl | H | OC(CH₃)₃ | H | H |
| 93 | H | Cl | OCH₃ | H | H |
| 94 | H | Cl | OC₂H₅ | H | H |
| 95 | H | Cl | OCH(CH₃)₂ | H | H |
| 96 | H | Cl | OC(CH₃)₃ | H | H |
| 97 | H | H | CHO | H | H |
| 98 | H | H | CN | H | H |
| 99 | H | H | NO₂ | H | H |
| 100 | H | NO₂ | H | H | H |
| 101 | H | H | C(O)OCH₃ | H | H |
| 102 | H | H | C(O)OC₂H₅ | H | H |
| 103 | H | H | C(O)OCH(CH₃)₂ | H | H |
| 104 | H | H | C(O)OC(CH₃)₃ | H | H |
| 105 | H | H | NH₂ | H | H |
| 106 | H | H | NHCH₃ | H | H |
| 107 | H | H | N(CH₃)₂ | H | H |
| 108 | H | H | NHCH₂CH₃ | H | H |
| 109 | H | H | N(CH₂CH₃)₂ | H | H |
| 110 | H | H | SCH₃ | H | H |
| 111 | H | H | SO₂CH₃ | H | H |
| 112 | H | H | SCH₂CH₃ | H | H |
| 113 | H | H | SO₂CH₂CH₃ | H | H |
| 114 | H | H | c-C₃H₅ | H | H |
| 115 | H | H | c-C₅H₉ | H | H |
| 116 | H | H | c-C₆H₁₁ | H | H |
| 117 | H | O-CH₂-O | | H | H |
| 118 | H | H | CH(=NOCH₃) | H | H |
| 119 | H | H | C(CH₃)(=NOCH₃) | H | H |
| 120 | H | SCH₃ | H | H | H |
| 121 | H | H | OCH₂CH₂CH₃ | H | H |
| 122 | H | H | C₆H₅O | H | H |
| 123 | H | H | C(O)CH₃ | H | H |
| 124 | H | H | C(CH₃)(=NOC₂H₅) | H | H |
| 125 | OC₂H₅ | H | H | H | H |
| 126 | CH₃ | CH₃ | H | H | H |
| 127 | CH₃ | H | F | H | H |
| 128 | OCH₃ | H | OCH₃ | H | H |
| 129 | F | H | H | F | H |
| 130 | CH₃ | H | H | CH₃ | H |
| 131 | H | OC₂H₅ | H | H | H |
| 132 | H | C(O)CH₃ | H | H | H |
| 133 | H | OCH(CH₃)₂ | H | H | H |
| 134 | H | C(CH₃)(=NOCH₃) | H | H | H |
| 135 | H | C(CH₃)(=NOC₂H₅) | H | H | H |
| 136 | H | CO₂CH₃ | H | H | H |
| 137 | H | CH₃ | CH₃ | H | H |
| 138 | H | OCH₃ | OCH₃ | H | H |
| 139 | H | CH₃ | Cl | H | H |
| 140 | H | CH₃ | OCH₃ | H | H |
| 141 | H | CH₃ | H | CH₃ | H |
| 142 | H | CF₃ | H | CF₃ | H |
| 143 | H | F | F | F | H |
| 144 | H | H | OCH₂CH₂CH₂CH₃ | H | H |

Eine besonders bevorzugte Ausführungsform der Erfindung sind die Verbindungen der allgemeinen Formel la, 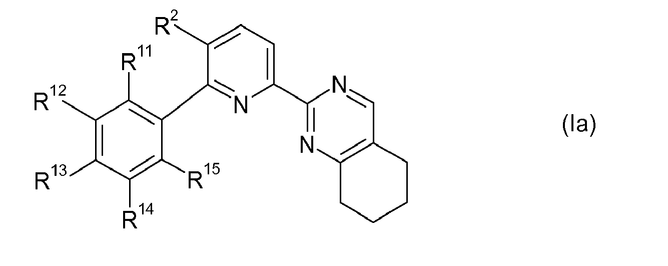 worin R¹¹, R¹², R¹³, R¹⁴ und R¹⁵ die zuvor angegebenen Bedeutungen und insbesondere die als bevorzugt angegebenen Bedeutungen aufweisen und R² die zuvor angegebenen, von H und C₁-C₄-Alkyl verschiedenen Bedeutungen aufweist und insbesondere für C₁-C₄-Alkoxy, speziell Methoxy, Ethoxy, Isopropyloxy, tert.-Butyloxy, C₁-C₂-Fluoralkyloxy, speziell Difluormethoxy oder Trifluormethoxy, CN, NO₂ oder OH steht. Beispiele hierfür sind die in den folgenden Tabellen 1 bis 9 angegebenen Verbindungen der Formel la worin R¹¹, R¹², R¹³, R¹⁴ und R¹⁵ gemeinsam die in einer der Zeilen 2 bis 144 der Tabelle A angegebenen Bedeutung besitzen.
Tabelle 1:
Verbindungen der allgemeinen Formel Ia, worin R² für Methoxy steht und R¹¹, R¹², R¹³, R¹⁴ und R¹⁵ gemeinsam die in einer der Zeilen 2 bis 144 der Tabelle A angegebenen Bedeutung besitzen.
Tabelle 2:
Verbindungen der allgemeinen Formel Ia, worin R² für Ethoxy steht und R¹¹, R¹², R¹³, R¹⁴ und R¹⁵ gemeinsam die in einer der Zeilen 2 bis 144 der Tabelle A angegebenen Bedeutung besitzen.
Tabelle 3:
Verbindungen der allgemeinen Formel Ia, worin R² für Isopropyloxy steht und R¹¹, R¹², R¹³, R¹⁴ und R¹⁵ gemeinsam die in einer der Zeilen 2 bis 144 der Tabelle A angegebenen Bedeutung besitzen.
Tabelle 4:
Verbindungen der allgemeinen Formel Ia, worin R² für tert.-Butyloxy steht und R¹¹, R¹², R¹³, R¹⁴ und R¹⁵ gemeinsam die in einer der Zeilen 2 bis 144 der Tabelle A angegebenen Bedeutung besitzen.
Tabelle 5:
Verbindungen der allgemeinen Formel Ia, worin R² für Trifluormethoxy steht und R¹¹, R¹², R¹³, R¹⁴ und R¹⁵ gemeinsam die in einer der Zeilen 2 bis 144 der Tabelle A angegebenen Bedeutung besitzen.
Tabelle 6:
Verbindungen der allgemeinen Formel Ia, worin R² für Difluormethoxy steht und R¹¹, R¹², R¹³, R¹⁴ und R¹⁵ gemeinsam die in einer der Zeilen 2 bis 144 der Tabelle A angegebenen Bedeutung besitzen.
Tabelle 7:
Verbindungen der allgemeinen Formel Ia, worin R² für CN steht und R¹¹, R¹², R¹³, R¹⁴ und R¹⁵ gemeinsam die in einer der Zeilen 2 bis 144 der Tabelle A angegebenen Bedeutung besitzen.
Tabelle 8:
Verbindungen der allgemeinen Formel Ia, worin R² für Nitro steht und R¹¹, R¹², R¹³, R¹⁴ und R¹⁵ gemeinsam die in einer der Zeilen 2 bis 144 der Tabelle A angegebenen Bedeutung besitzen.
Tabelle 9:
Verbindungen der allgemeinen Formel Ia, worin R² für OH steht und R¹¹, R¹², R¹³, R¹⁴ und R¹⁵ gemeinsam die in einer der Zeilen 2 bis 144 der Tabelle A angegebenen Bedeutung besitzen.

Eine weitere besonders bevorzugte Ausführungsform der Erfindung sind die Verbindungen der allgemeinen Formel Ib 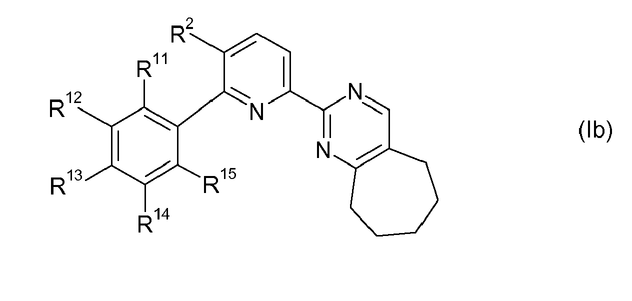 worin R¹¹, R¹², R¹³, R¹⁴ und R¹⁵ die zuvor angegebenen Bedeutungen und insbesondere die als bevorzugt angegebenen Bedeutungen aufweisen und R² die zuvor angegebenen Bedeutungen aufweist und insbesondere für C₁-C₄-Alkyl, speziell Methyl, Ethyl, n-Propyl, iso-Propyl, n-Butyl, 2-Butyl oder tert.-Butyl, C₁-C₄-Alkoxy, speziell Methoxy, Ethoxy, Isopropyloxy oder tert.-Butyloxy, C₁-C₂-Fluoralkyl, speziell Trifluormethyl oder Pentafluorethyl, C₁-C₂-Fluoralkyloxy, speziell Difluormethoxy oder Trifluormethoxy, Halogen, speziell Fluor, Chlor oder Brom, CN, NO₂ oder OH steht. Beispiele hierfür sind die in den folgenden Tabellen 10 bis 30 angegebenen Verbindungen der Formel lb, worin R¹¹, R¹², R¹³, R¹⁴ und R¹⁵ gemeinsam die in einer der Zeilen 1 bis 144 der Tabelle A angegebenen Bedeutung besitzen.
Tabelle 10:
Verbindungen der allgemeinen Formel Ib, worin R² für Methyl steht und R¹¹, R¹², R¹³, R¹⁴ und R¹⁵ gemeinsam die in einer der Zeilen 1 bis 144 der Tabelle A angegebenen Bedeutung besitzen.
Tabelle 11:
Verbindungen der allgemeinen Formel Ib, worin R² für Ethyl steht und R¹¹, R¹², R¹³, R¹⁴ und R¹⁵ gemeinsam die in einer der Zeilen 1 bis 144 der Tabelle A angegebenen Bedeutung besitzen.
Tabelle 12:
Verbindungen der allgemeinen Formel Ib, worin R² für n-Propyl steht und R¹¹, R¹², R¹³, R¹⁴ und R¹⁵ gemeinsam die in einer der Zeilen 1 bis 144 der Tabelle A angegebenen Bedeutung besitzen.
Tabelle 13:
Verbindungen der allgemeinen Formel Ib, worin R² für Isopropyl steht und R¹¹, R¹², R¹³, R¹⁴ und R¹⁵ gemeinsam die in einer der Zeilen 1 bis 144 der Tabelle A angegebenen Bedeutung besitzen.
Tabelle 14:
Verbindungen der allgemeinen Formel Ib, worin R² für n-Butyl steht und R¹¹, R¹², R¹³, R¹⁴ und R¹⁵ gemeinsam die in einer der Zeilen 1 bis 144 der Tabelle A angegebenen Bedeutung besitzen.
Tabelle 15:
Verbindungen der allgemeinen Formel Ib, worin R² für 2-Butyl steht und R¹¹, R¹², R¹³, R¹⁴ und R¹⁵ gemeinsam die in einer der Zeilen 1 bis 144 der Tabelle A angegebenen Bedeutung besitzen.
Tabelle 16:
Verbindungen der allgemeinen Formel Ib, worin R² für tert.-Butyl steht und R¹¹, R¹², R¹³, R¹⁴ und R¹⁵ gemeinsam die in einer der Zeilen 1 bis 144 der Tabelle A angegebenen Bedeutung besitzen.
Tabelle 17:
Verbindungen der allgemeinen Formel Ib, worin R² für Methoxy steht und R¹¹, R¹², R¹³, R¹⁴ und R¹⁵ gemeinsam die in einer der Zeilen 1 bis 144 der Tabelle A angegebenen Bedeutung besitzen.
Tabelle 18:
Verbindungen der allgemeinen Formel Ib, worin R² für Ethoxy steht und R¹¹, R¹², R¹³, R¹⁴ und R¹⁵ gemeinsam die in einer der Zeilen 1 bis 144 der Tabelle A angegebenen Bedeutung besitzen.
Tabelle 19:
Verbindungen der allgemeinen Formel Ib, worin R² für Isopropyloxy steht und R¹¹, R¹², R¹³, R¹⁴ und R¹⁵ gemeinsam die in einer der Zeilen 1 bis 144 der Tabelle A angegebenen Bedeutung besitzen.
Tabelle 20:
Verbindungen der allgemeinen Formel Ib, worin R² für tert.-Butyloxy steht und R¹¹, R¹², R¹³, R¹⁴ und R¹⁵ gemeinsam die in einer der Zeilen 1 bis 144 der Tabelle A angegebenen Bedeutung besitzen.
Tabelle 21:
Verbindungen der allgemeinen Formel Ib, worin R² für Trifluormethoxy steht und R¹¹, R¹², R¹³, R¹⁴ und R¹⁵ gemeinsam die in einer der Zeilen 1 bis 144 der Tabelle A angegebenen Bedeutung besitzen.
Tabelle 22:
Verbindungen der allgemeinen Formel Ib, worin R² für Difluormethoxy steht und R¹¹, R¹², R¹³, R¹⁴ und R¹⁵ gemeinsam die in einer der Zeilen 1 bis 144 der Tabelle A angegebenen Bedeutung besitzen.
Tabelle 23:
Verbindungen der allgemeinen Formel Ib, worin R² für Trifluormethyl steht und R¹¹, R¹², R¹³, R¹⁴ und R¹⁵ gemeinsam die in einer der Zeilen 1 bis 144 der Tabelle A angegebenen Bedeutung besitzen.
Tabelle 24:
Verbindungen der allgemeinen Formel Ib, worin R² für Pentafluorethyl steht und R¹¹, R¹², R¹³, R¹⁴ und R¹⁵ gemeinsam die in einer der Zeilen 1 bis 144 der Tabelle A angegebenen Bedeutung besitzen.
Tabelle 25:
Verbindungen der allgemeinen Formel Ib, worin R² für Fluor steht und R¹¹, R¹², R¹³, R¹⁴ und R¹⁵ gemeinsam die in einer der Zeilen 1 bis 144 der Tabelle A angegebenen Bedeutung besitzen.
Tabelle 26:
Verbindungen der allgemeinen Formel Ib, worin R² für Chlor steht und R¹¹, R¹², R¹³, R¹⁴ und R¹⁵ gemeinsam die in einer der Zeilen 1 bis 144 der Tabelle A angegebenen Bedeutung besitzen.
Tabelle 27:
Verbindungen der allgemeinen Formel Ib, worin R² für Brom steht und R¹¹, R¹², R¹³, R¹⁴ und R¹⁵ gemeinsam die in einer der Zeilen 1 bis 144 der Tabelle A angegebenen Bedeutung besitzen.
Tabelle 28:
Verbindungen der allgemeinen Formel Ib, worin R² für CN steht und R¹¹, R¹², R¹³, R¹⁴ und R¹⁵ gemeinsam die in einer der Zeilen 1 bis 144 der Tabelle A angegebenen Bedeutung besitzen.
Tabelle 29:
Verbindungen der allgemeinen Formel Ib, worin R² für NO₂ steht und R¹¹, R¹², R¹³, R¹⁴ und R¹⁵ gemeinsam die in einer der Zeilen 1 bis 144 der Tabelle A angegebenen Bedeutung besitzen.
Tabelle 30:
Verbindungen der allgemeinen Formel Ib, worin R² für OH steht und R¹¹, R¹², R¹³, R¹⁴ und R¹⁵ gemeinsam die in einer der Zeilen 1 bis 144 der Tabelle A angegebenen Bedeutung besitzen.

Eine weitere, besonders bevorzugte Ausführungsform der Erfindung sind die Verbindungen der allgemeinen Formel Ic 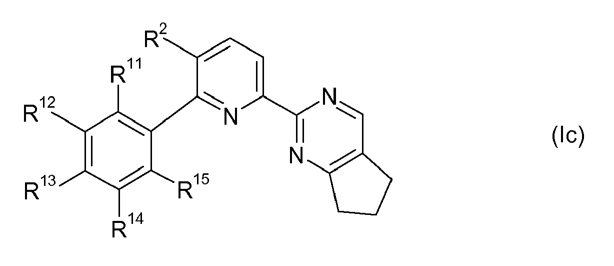 worin R¹¹, R¹², R¹³, R¹⁴ und R¹⁵ die zuvor angegebenen Bedeutungen und insbesondere die als bevorzugt angegebenen Bedeutungen aufweisen und R² die zuvor angegebenen, von H und C₁-C₄-Alkyl verschiedenen Bedeutungen aufweist und insbesondere für C₁-C₄-Alkoxy, speziell Methoxy, Ethoxy, Isopropyloxy, tert.-Butyloxy, C₁-C₂-Fluoralkyloxy, speziell Difluormethoxy oder Trifluormethoxy, CN, NO₂ oder OH steht. Beispiele hierfür sind die in den folgenden Tabellen 31 bis 39 angegebenen Verbindungen der Formel Ic, worin R¹¹, R¹², R¹³, R¹⁴ und R¹⁵ gemeinsam die in einer der Zeilen 2 bis 144 der Tabelle A angegebenen Bedeutung besitzen.
Tabelle 31:
Verbindungen der allgemeinen Formel Ic, worin R² für Methoxy steht und R¹¹, R¹², R¹³, R¹⁴ und R¹⁵ gemeinsam die in einer der Zeilen 2 bis 144 der Tabelle A angegebenen Bedeutung besitzen.
Tabelle 32:
Verbindungen der allgemeinen Formel Ic, worin R² für Ethoxy steht und R¹¹, R¹², R¹³, R¹⁴ und R¹⁵ gemeinsam die in einer der Zeilen 2 bis 144 der Tabelle A angegebenen Bedeutung besitzen.
Tabelle 33:
Verbindungen der allgemeinen Formel Ic, worin R² für Isopropyloxy steht und R¹¹, R¹², R¹³, R¹⁴ und R¹⁵ gemeinsam die in einer der Zeilen 2 bis 144 der Tabelle A angegebenen Bedeutung besitzen.
Tabelle 34:
Verbindungen der allgemeinen Formel Ic, worin R² für tert.-Butyloxy steht und R¹¹, R¹², R¹³, R¹⁴ und R¹⁵ gemeinsam die in einer der Zeilen 2 bis 144 der Tabelle A angegebenen Bedeutung besitzen.
Tabelle 35:
Verbindungen der allgemeinen Formel Ic, worin R² für Trifluormethoxy steht und R¹¹, R¹², R¹³, R¹⁴ und R¹⁵ gemeinsam die in einer der Zeilen 2 bis 144 der Tabelle A angegebenen Bedeutung besitzen.
Tabelle 36:
Verbindungen der allgemeinen Formel Ic, worin R² für Difluormethoxy steht und R¹¹, R¹², R¹³, R¹⁴ und R¹⁵ gemeinsam die in einer der Zeilen 2 bis 144 der Tabelle A angegebenen Bedeutung besitzen.
Tabelle 37:
Verbindungen der allgemeinen Formel Ic, worin R² für CN steht und R¹¹, R¹², R¹³, R¹⁴ und R¹⁵ gemeinsam die in einer der Zeilen 2 bis 144 der Tabelle A angegebenen Bedeutung besitzen.
Tabelle 38:
Verbindungen der allgemeinen Formel Ic, worin R² für Nitro steht und R¹¹, R¹², R¹³, R¹⁴ und R¹⁵ gemeinsam die in einer der Zeilen 2 bis 144 der Tabelle A angegebenen Bedeutung besitzen.
Tabelle 39:
Verbindungen der allgemeinen Formel Ic, worin R² für OH steht und R¹¹, R¹², R¹³, R¹⁴ und R¹⁵ gemeinsam die in einer der Zeilen 2 bis 144 der Tabelle A angegebenen Bedeutung besitzen.

Eine weitere besonders bevorzugte Ausführungsform der Erfindung sind die Verbindungen der allgemeinen Formel Id 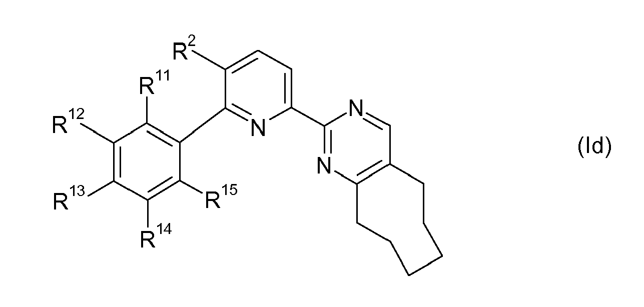 worin R¹¹, R¹², R¹³, R¹⁴ und R¹⁵ die zuvor angegebenen Bedeutungen und insbesondere die als bevorzugt angegebenen Bedeutungen aufweisen und R² die zuvor angegebenen Bedeutungen aufweist und insbesondere für C₁-C₄-Alkyl, speziell Methyl, Ethyl, n-Propyl, iso-Propyl, n-Butyl, 2-Butyl oder tert.-Butyl, C₁-C₄-Alkoxy, speziell Methoxy, Ethoxy, Isopropyloxy oder tert.-Butyloxy, C₁-C₂-Fluoralkyl, speziell Trifluormethyl oder Pentafluorethyl, C₁-C₂-Fluoralkyloxy, speziell Difluormethoxy oder Trifluormethoxy, Halogen, speziell Fluor, Chlor oder Brom, CN, NO₂ oder OH steht. Beispiele hierfür sind die in den folgenden Tabellen 40 bis 60 angegebenen Verbindungen der Formel Id, worin R¹¹, R¹², R¹³, R¹⁴ und R¹⁵ gemeinsam die in einer der Zeilen 1 bis 144 der Tabelle A angegebenen Bedeutung besitzen.
Tabelle 40:
Verbindungen der allgemeinen Formel Id, worin R² für Methyl steht und R¹¹, R¹², R¹³, R¹⁴ und R¹⁵ gemeinsam die in einer der Zeilen 1 bis 144 der Tabelle A angegebenen Bedeutung besitzen.
Tabelle 41:
Verbindungen der allgemeinen Formel Id, worin R² für Ethyl steht und R¹¹, R¹², R¹³, R¹⁴ und R¹⁵ gemeinsam die in einer der Zeilen 1 bis 144 der Tabelle A angegebenen Bedeutung besitzen.
Tabelle 42:
Verbindungen der allgemeinen Formel Id, worin R² für n-Propyl steht und R¹¹, R¹² R¹³, R¹⁴ und R¹⁵ gemeinsam die in einer der Zeilen 1 bis 144 der Tabelle A angegebenen Bedeutung besitzen.
Tabelle 43:
Verbindungen der allgemeinen Formel Id, worin R² für Isopropyl steht und R¹¹, R¹², R¹³, R¹⁴ und R¹⁵ gemeinsam die in einer der Zeilen 1 bis 144 der Tabelle A angegebenen Bedeutung besitzen.
Tabelle 44:
Verbindungen der allgemeinen Formel Id, worin R² für n-Butyl steht und R¹¹, R¹² R¹³, R¹⁴ und R¹⁵ gemeinsam die in einer der Zeilen 1 bis 144 der Tabelle A angegebenen Bedeutung besitzen.
Tabelle 45:
Verbindungen der allgemeinen Formel Id, worin R² für 2-Butyl steht und R¹¹, R¹² R¹³, R¹⁴ und R¹⁵ gemeinsam die in einer der Zeilen 1 bis 144 der Tabelle A angegebenen Bedeutung besitzen.
Tabelle 46:
Verbindungen der allgemeinen Formel Id, worin R² für tert.-Butyl steht und R¹¹, R¹², R¹³, R¹⁴ und R¹⁵ gemeinsam die in einer der Zeilen 1 bis 144 der Tabelle A angegebenen Bedeutung besitzen.
Tabelle 47:
Verbindungen der allgemeinen Formel Id, worin R² für Methoxy steht und R¹¹, R¹², R¹³, R¹⁴ und R¹⁵ gemeinsam die in einer der Zeilen 1 bis 144 der Tabelle A angegebenen Bedeutung besitzen.
Tabelle 48:
Verbindungen der allgemeinen Formel Id, worin R² für Ethoxy steht und R¹¹, R¹², R¹³, R¹⁴ und R¹⁵ gemeinsam die in einer der Zeilen 1 bis 144 der Tabelle A angegebenen Bedeutung besitzen.
Tabelle 49:
Verbindungen der allgemeinen Formel Id, worin R² für Isopropyloxy steht und R¹¹, R¹², R¹³, R¹⁴ und R¹⁵ gemeinsam die in einer der Zeilen 1 bis 144 der Tabelle A angegebenen Bedeutung besitzen.
Tabelle 50:
Verbindungen der allgemeinen Formel Id, worin R² für tert.-Butyloxy steht und R¹¹, R¹², R¹³, R¹⁴ und R¹⁵ gemeinsam die in einer der Zeilen 1 bis 144 der Tabelle A angegebenen Bedeutung besitzen.
Tabelle 51:
Verbindungen der allgemeinen Formel Id, worin R² für Trifluormethoxy steht und R¹¹, R¹², R¹³, R¹⁴ und R¹⁵ gemeinsam die in einer der Zeilen 1 bis 144 der Tabelle A angegebenen Bedeutung besitzen.
Tabelle 52:
Verbindungen der allgemeinen Formel Id, worin R² für Difluormethoxy steht und R¹¹, R¹², R¹³, R¹⁴ und R¹⁵ gemeinsam die in einer der Zeilen 1 bis 144 der Tabelle A angegebenen Bedeutung besitzen.
Tabelle 53:
Verbindungen der allgemeinen Formel Id, worin R² für Trifluormethyl steht und R¹¹, R¹², R¹³, R¹⁴ und R¹⁵ gemeinsam die in einer der Zeilen 1 bis 144 der Tabelle A angegebenen Bedeutung besitzen.
Tabelle 54:
Verbindungen der allgemeinen Formel Id, worin R² für Pentafluorethyl steht und R¹¹, R¹², R¹³, R¹⁴ und R¹⁵ gemeinsam die in einer der Zeilen 1 bis 144 der Tabelle A angegebenen Bedeutung besitzen.
Tabelle 55:
Verbindungen der allgemeinen Formel Id, worin R² für Fluor steht und R¹¹, R¹², R¹³, R¹⁴ und R¹⁵ gemeinsam die in einer der Zeilen 1 bis 144 der Tabelle A angegebenen Bedeutung besitzen.
Tabelle 56:
Verbindungen der allgemeinen Formel Id, worin R² für Chlor steht und R¹¹, R¹², R¹³, R¹⁴ und R¹⁵ gemeinsam die in einer der Zeilen 1 bis 144 der Tabelle A angegebenen Bedeutung besitzen.
Tabelle 57:
Verbindungen der allgemeinen Formel Id, worin R² für Brom steht und R¹¹, R¹², R¹³, R¹⁴ und R¹⁵ gemeinsam die in einer der Zeilen 1 bis 144 der Tabelle A angegebenen Bedeutung besitzen.
Tabelle 58:
Verbindungen der allgemeinen Formel Id, worin R² für CN steht und R¹¹, R¹², R¹³, R¹⁴ und R¹⁵ gemeinsam die in einer der Zeilen 1 bis 144 der Tabelle A angegebenen Bedeutung besitzen.
Tabelle 59:
Verbindungen der allgemeinen Formel Id, worin R² für NO₂ steht und R¹¹, R¹², R¹³, R¹⁴ und R¹⁵ gemeinsam die in einer der Zeilen 1 bis 144 der Tabelle A angegebenen Bedeutung besitzen.
Tabelle 60:
Verbindungen der allgemeinen Formel Id, worin R² für OH steht und R¹¹, R¹², R¹³, R¹⁴ und R¹⁵ gemeinsam die in einer der Zeilen 1 bis 144 der Tabelle A angegebenen Bedeutung besitzen.

Eine weitere bevorzugte Ausführungsform der Erfindung sind die Verbindungen der allgemeinen Formel Ie, 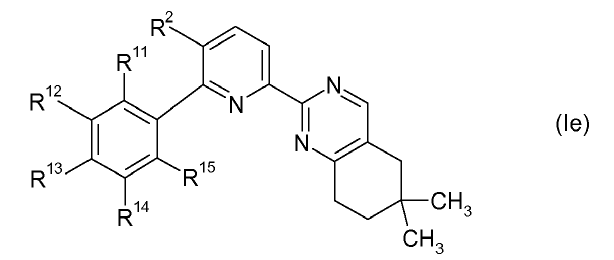 worin R¹¹, R¹², R¹³, R¹⁴ und R¹⁵ die zuvor angegebenen Bedeutungen und insbesondere die als bevorzugt angegebenen Bedeutungen aufweisen und R² die zuvor angegebenen Bedeutungen aufweist und insbesondere für C₁-C₄-Alkyl, speziell Methyl, Ethyl, n-Propyl, iso-Propyl, n-Butyl, 2-Butyl oder tert.-Butyl, C₁-C₄-Alkoxy, speziell Methoxy, Ethoxy, Isopropyloxy oder tert.-Butyloxy, C₁-C₂-Fluoralkyl, speziell Trifluormethyl oder Pentafluorethyl, C₁-C₂-Fluoralkyloxy, speziell Difluormethoxy oder Trifluormethoxy, Halogen, speziell Fluor, Chlor oder Brom, CN, NO₂ oder OH steht. Beispiele hierfür sind die in den folgenden Tabellen 61 bis 81 angegebenen Verbindungen der Formel Ie, worin R¹¹, R¹², R¹³, R¹⁴ und R¹⁵ gemeinsam die in einer der Zeilen 1 bis 144 der Tabelle A angegebenen Bedeutung besitzen.
Tabelle 61:
Verbindungen der allgemeinen Formel Ie, worin R² für Methyl steht und R¹¹, R¹², R¹³, R¹⁴ und R¹⁵ gemeinsam die in einer der Zeilen 1 bis 144 der Tabelle A angegebenen Bedeutung besitzen.
Tabelle 62:
Verbindungen der allgemeinen Formel Ie, worin R² für Ethyl steht und R¹¹, R¹², R¹³, R¹⁴ und R¹⁵ gemeinsam die in einer der Zeilen 1 bis 144 der Tabelle A angegebenen Bedeutung besitzen.
Tabelle 63:
Verbindungen der allgemeinen Formel Ie, worin R² für n-Propyl steht und R¹¹, R¹², R¹³, R¹⁴ und R¹⁵ gemeinsam die in einer der Zeilen 1 bis 144 der Tabelle A angegebenen Bedeutung besitzen.
Tabelle 64:
Verbindungen der allgemeinen Formel Ie, worin R² für Isopropyl steht und R¹¹, R¹², R¹³, R¹⁴ und R¹⁵ gemeinsam die in einer der Zeilen 1 bis 144 der Tabelle A angegebenen Bedeutung besitzen.
Tabelle 65:
Verbindungen der allgemeinen Formel Ie, worin R² für n-Butyl steht und R¹¹, R¹², R¹³, R¹⁴ und R¹⁵ gemeinsam die in einer der Zeilen 1 bis 144 der Tabelle A angegebenen Bedeutung besitzen.
Tabelle 66:
Verbindungen der allgemeinen Formel Ie, worin R² für 2-Butyl steht und R¹¹, R¹², R¹³, R¹⁴ und R¹⁵ gemeinsam die in einer der Zeilen 1 bis 144 der Tabelle A angegebenen Bedeutung besitzen.
Tabelle 67:
Verbindungen der allgemeinen Formel Ie, worin R² für tert.-Butyl steht und R¹¹, R¹², R¹³, R¹⁴ und R¹⁵ gemeinsam die in einer der Zeilen 1 bis 144 der Tabelle A angegebenen Bedeutung besitzen.
Tabelle 68:
Verbindungen der allgemeinen Formel Ie, worin R² für Methoxy steht und R¹¹, R¹², R¹³, R¹⁴ und R¹⁵ gemeinsam die in einer der Zeilen 1 bis 144 der Tabelle A angegebenen Bedeutung besitzen.
Tabelle 69:
Verbindungen der allgemeinen Formel Ie, worin R² für Ethoxy steht und R¹¹, R¹², R¹³, R¹⁴ und R¹⁵ gemeinsam die in einer der Zeilen 1 bis 144 der Tabelle A angegebenen Bedeutung besitzen.
Tabelle 70:
Verbindungen der allgemeinen Formel Ie, worin R² für Isopropyloxy steht und R¹¹, R¹², R¹³, R¹⁴ und R¹⁵ gemeinsam die in einer der Zeilen 1 bis 144 der Tabelle A angegebenen Bedeutung besitzen.
Tabelle 71:
Verbindungen der allgemeinen Formel Ie, worin R² für tert.-Butyloxy steht und R¹¹, R¹², R¹³, R¹⁴ und R¹⁵ gemeinsam die in einer der Zeilen 1 bis 144 der Tabelle A angegebenen Bedeutung besitzen.
Tabelle 72:
Verbindungen der allgemeinen Formel Ie, worin R² für Trifluormethoxy steht und R¹¹, R¹², R¹³, R¹⁴ und R¹⁵ gemeinsam die in einer der Zeilen 1 bis 144 der Tabelle A angegebenen Bedeutung besitzen.
Tabelle 73:
Verbindungen der allgemeinen Formel Ie, worin R² für Difluormethoxy steht und R¹¹, R¹², R¹³, R¹⁴ und R¹⁵ gemeinsam die in einer der Zeilen 1 bis 144 der Tabelle A angegebenen Bedeutung besitzen.
Tabelle 74:
Verbindungen der allgemeinen Formel Ie, worin R² für Trifluormethyl steht und R¹¹, R¹², R¹³, R¹⁴ und R¹⁵ gemeinsam die in einer der Zeilen 1 bis 144 der Tabelle A angegebenen Bedeutung besitzen.
Tabelle 75:
Verbindungen der allgemeinen Formel Ie, worin R² für Pentafluorethyl steht und R¹¹, R¹², R¹³, R¹⁴ und R¹⁵ gemeinsam die in einer der Zeilen 1 bis 144 der Tabelle A angegebenen Bedeutung besitzen.
Tabelle 76:
Verbindungen der allgemeinen Formel Ie, worin R² für Fluor steht und R¹¹, R¹², R¹³, R¹⁴ und R¹⁵ gemeinsam die in einer der Zeilen 1 bis 144 der Tabelle A angegebenen Bedeutung besitzen.
Tabelle 77:
Verbindungen der allgemeinen Formel Ie, worin R² für Chlor steht und R¹¹, R¹², R¹³, R¹⁴ und R¹⁵ gemeinsam die in einer der Zeilen 1 bis 144 der Tabelle A angegebenen Bedeutung besitzen.
Tabelle 78:
Verbindungen der allgemeinen Formel Ie, worin R² für Brom steht und R¹¹, R¹², R¹³, R¹⁴ und R¹⁵ gemeinsam die in einer der Zeilen 1 bis 144 der Tabelle A angegebenen Bedeutung besitzen.
Tabelle 79:
Verbindungen der allgemeinen Formel Ie, worin R² für CN steht und R¹¹, R¹², R¹³, R¹⁴ und R¹⁵ gemeinsam die in einer der Zeilen 1 bis 144 der Tabelle A angegebenen Bedeutung besitzen.
Tabelle 80:
Verbindungen der allgemeinen Formel Ie, worin R² für NO₂ steht und R¹¹, R¹², R¹³, R¹⁴ und R¹⁵ gemeinsam die in einer der Zeilen 1 bis 144 der Tabelle A angegebenen Bedeutung besitzen.
Tabelle 81:
Verbindungen der allgemeinen Formel Ie, worin R² für OH steht und R¹¹, R¹², R¹³, R¹⁴ und R¹⁵ gemeinsam die in einer der Zeilen 1 bis 144 der Tabelle A angegebenen Bedeutung besitzen.

Die erfindungsgemäßen Verbindungen der allgemeinen Formel I können beispielsweise nach dem in Schema 1 dargestellten Verfahren hergestellt werden:

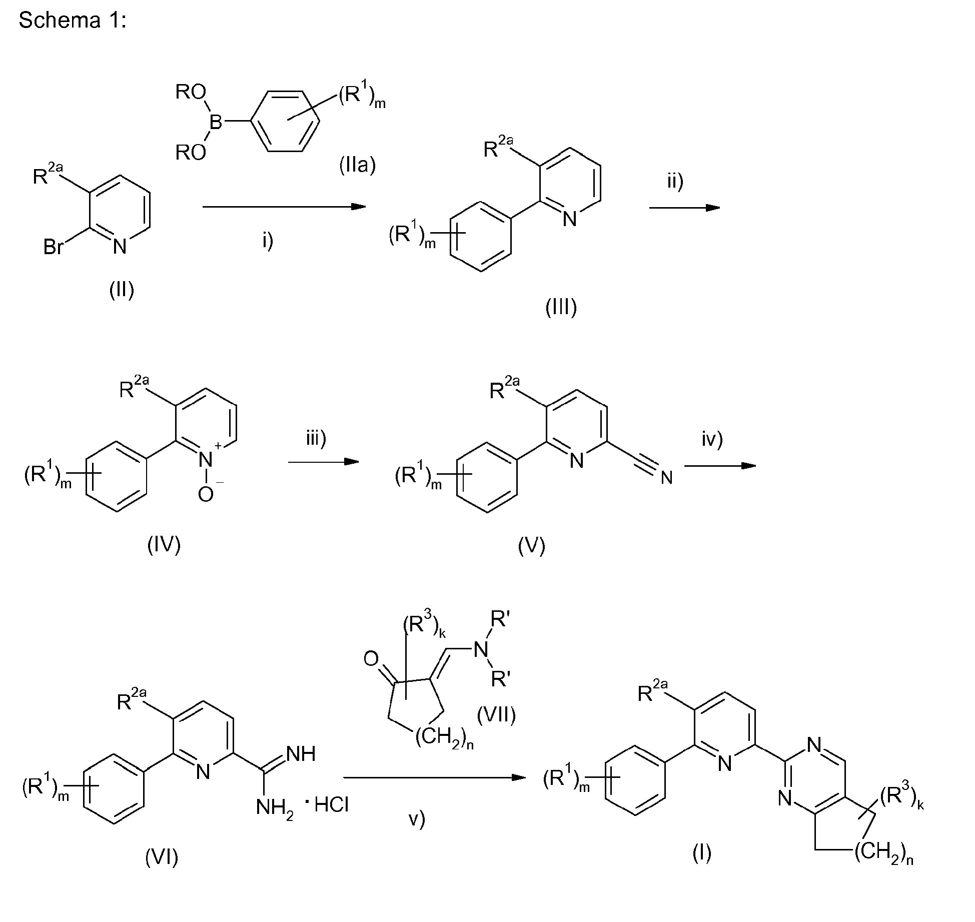

In Schema 1 haben R¹, R³, m, n und k die zuvor genannten Bedeutungen. R^{2a} steht für H, C₁-C₄-Alkyl, C₁-C₄-Halogenalkyl, Halogen, C₁-C₄-Alkoxy, C-C4-Halogenalkoxy, NO₂ oder OH. R steht für H oder C₁-C₄-Alkyl oder bildet mit weiteren Molekülen IIa ein Phenylboronsäure-Anhydrid. R' steht für C₁-C₄-Alkyl und insbesondere für Methyl.

In einem ersten Schritt i) wird ein 2-Brompyridin mit einem Phenylboronsäurederivat der allgemeinen Formel IIa unter den Bedingungen einer Suzuki-Kupplung, d.h. in Gegenwart eine Platinmetall-Katalysators und insbesondere in Gegenwart eines Palladiumkatalysators unter an sich bekannten Reaktionsbedingungen umgesetzt, wie sie z.B. aus Acc. Chem. Res. 15, S 178-184 (1982), Chem. Rev. 95, S. 2457-2483 (1995), und der darin zitierten Literatur, sowie aus J. Org. Chem. 68, S. 9412 (2003) bekannt sind. Geeignete Katalysatoren sind insbesondere Tetrakis(triphenylphosphin)palladium(0), Bis(triphenylphosphin)palladium(II)-chlorid, Bis(acetonitril)palladium(II)-chlorid, der [1,1'-Bis-(diphenylphosphino)ferrocen]-palladium(II)-chlorid-Dichlormethan-Komplex, Bis-[1,2-bis(diphenylphosphin)ethan]palladium(0) und [1,4-Bis(diphenyl-phosphin)butan]palladium(II)-chlorid. Die Menge an Katalysator beträgt üblicherweise 0,1 bis 10 mol-%.

Anschließend wird das so erhaltene 2-Phenylpyridin der Formel III in Schritt ii) durch Behandlung mit einer Persäure nach an sich bekannten Methoden in das 2-Phenylpyridin-N-oxid der Formel IV umgewandelt. Die Umwandlung von III in IV kann in Analogie zu bekannten Verfahren erfolgen, beispielsweise durch Behandlung von III mit Wasserstoffperoxid in einer organischen Säure wie Ameisensäure, Essigsäure, Chloressigsäure oder Trifluoressigsäure (siehe z. B. J. Org. Chem. 55, S. 738-741 (1990) und Organic Synthesis, Collect. Vol. IV, S. 655-656 (1963)) oder durch Umsetzung von III mit einer organischen Persäure wie meta-Chlorperbenzoesäure in einem inerten Lösungsmittel, z. B. einem halogenierten Kohlenwasserstoff wie Dichlormethan oder Dichlorethan (siehe z. B. Synthetic Commun. 22(18), S. 2645, (1992); J. Med. Chem. 2146 (1998)). Die Umwandlung von III nach IV gelingt auch in Analogie zu der von K. B. Sharpless (J. Org. Chem. 63(5), S. 7740 (1998)) beschriebenen Methode durch Umsetzung von III mit Wasserstoffperoxid in einem halogenierten Kohlenwasserstoff wie Dichlormethan oder Dichlorethan in Gegenwart katalytischer Mengen (z. B. 5 Gew.-%) Rhenium(VII)-Verbindungen wie Methyltrioxorhenium (H₃CReO₃).

Die Umwandlung des 2-Phenylpyridin-N-oxids der Formel IV in das Nitril V in Schritt iii) kann beispielsweise durch eine modifizierte Reissert-Henze-Reaktion in Analogie zu den in J. Org. Chem. 48, S. 1375 (1983) und J. Org. Chem. 55, S. 738-741 (1990) beschriebenen Methoden durch Umsetzung von IV mit einem Trialkylsilylcyanid wie Trimethylsilylcyanid in Gegenwart von Chlorameisensäure-N,N-dimethylamid erfolgen. Alternativ kann das Nitril V aus dem 2-Phenylpyridin-N-oxid IV durch sukzessive Umsetzung mit Dimethylsulfat und anschließend mit Cyanidionen, z. B. mit Natrium- oder Kaliumcyanid in Analogie zu der in Tetrahedron S. 4947 (1985) beschriebenen Methode hergestellt werden.

Die in Schritt iii) erhaltene 2-Cyano-6-phenylpyridinverbindung der allgemeinen Formel V wird anschließend in Schritt iv) nach der in US 4,873,248 beschriebenen Methode in das Amidinium-Hydrochlorid der Formel VI umgewandelt. Die Umwandlung erfolgt durch sukzessive Behandlung mit Alkalimetallalkoholat wie Natriummethanolat oder
- ethanolat und anschließende Umsetzung mit Ammoniumchlorid. Anstelle der Hydrochloride können im Folgeschritt v) auch die Hydrobromide, Acetate, Sulfate oder Formiate eingesetzt werden.

Anschließend wird das so erhaltene Amidinium-Hydrochlorid der Formel VI in Schritt v) mit einem Dialkylaminomethylencycloalkanon der Formel VII (Enaminoketon VII) in Gegenwart einer Base, vorzugsweise einem Alkalimetallalkoholat wie Natriummethylat oder Natriumethylat umgesetzt. Die Umsetzung kann in Analogie zu bekannten Verfahren zur Umsetzung von Amidinium-Hydrochloriden mit Enaminoketonen erfolgen wie sie beispielsweise in J. Heterocycl. Chem. 20, S. 649-653 (1983) beschrieben werden.

Anstelle der Enaminoketone VII können in Schritt v) auch β-Oxoacetale der Formel Vlla eingesetzt werden

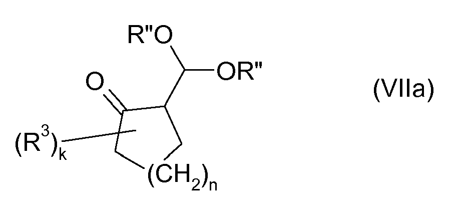

In Formel Vlla steht R" für C₁-C₄-Alkyl und insbesondere für Methyl oder Ethyl. Die Umsetzung VI mit Vlla kann in Analogie zu der in EP-A 259139 beschriebenen Methode (a) erfolgen, auf die hiermit Bezug genommen wird.

Dialkylaminomethylencycloalkanone der Formel VII sind bekannt oder können in Anlehnung an bekannte Methoden hergestellt werden [siehe z. B. WO 2001/087845, Tetrahedron 50(7), S. 2255-2264 (1994); Synthetic Communications 28(10), 1743-1753 (1998) oder Tetrahedron Letters 27(23), 2567-70 (1986)]. β-Oxoacetale der Formel Vlla sind ebenfalls bekannt, z. B. aus EP 259139, oder können kommerziell erworben werden.

Alternativ können die Verbindungen der Formel I nach der in Schema 2 dargestellten Syntheseroute hergestellt werden:

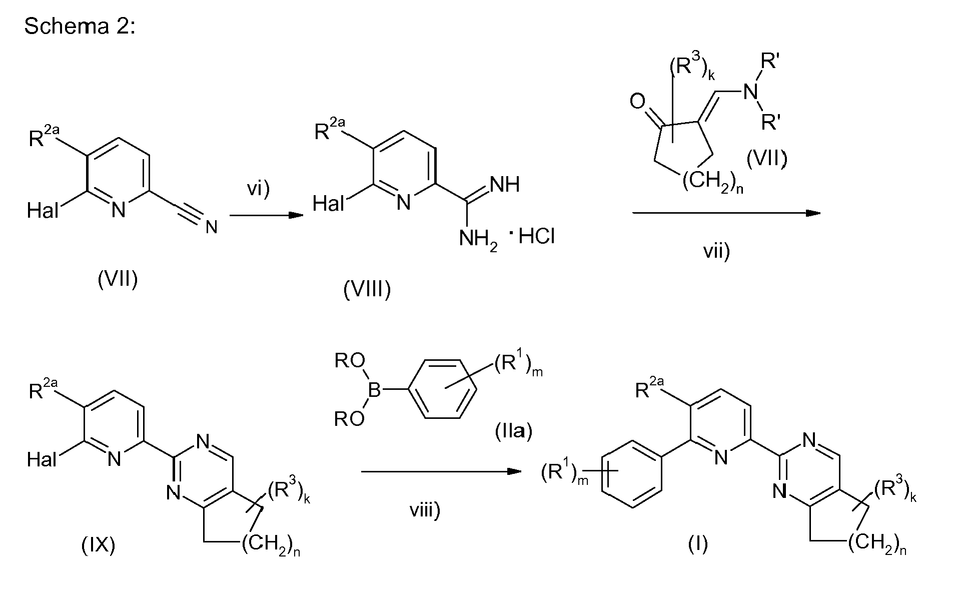

In Schema 2 haben R, R', R¹, R³, m, n und k die zuvor genannten Bedeutungen. R^{2a} steht für H, C₁-C₄-Alkyl oder C₁-C₄-Halogenalkyl. Hal steht für Brom oder Chlor.

Hinsichtlich der Reaktionsbedingungen für Schritt vi) gilt das für Schritt iv) in Schema 1 Gesagte. Hinsichtlich der Reaktionsbedingungen für Schritt vii) gilt das für Schritt v) in Schema 1 Gesagte. Hinsichtlich der Reaktionsbedingungen für Schritt viii) gilt das für Schritt i) in Schema 1 Gesagte. Cyanopyridine der Formel VII sind bekannt, z. B. aus US 2003/087940, WO 2004/026305, WO 01/057046 und Bioorg. Med. Chem. Lett. S. 1571-1574 (2003) oder können nach bekannten Herstellungsverfahren hergestellt werden.

Verbindungen der allgemeinen Formel VII können insbesondere nach dem in Schema 3 dargestellten Verfahren hergestellt werden.

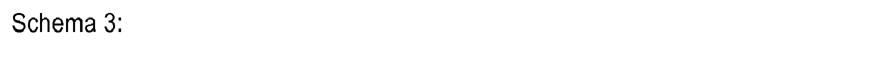

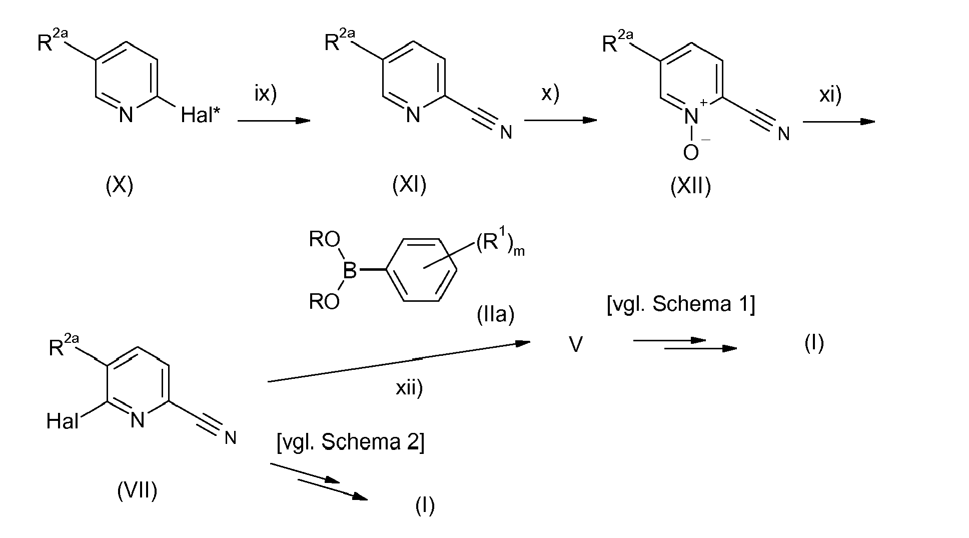

In Schema 3 haben R, R¹ und m die zuvor genannten Bedeutungen. R^{2a} steht für C₁-C₄-Alkyl, C₁-C₄-Halogenalkyl, Halogen, C₁-C₄-Alkoxy, C₁-C₄-Halogenalkoxy oder NO₂ . Hal und Hal* stehen unabhängig voneinander für Chlor oder Brom, wobei Hal* auch Iod bedeuten kann.

Die Umwandlung des 2-Halogenpyridins X in das 2-Cyanopyridin XI in Schritt ix) gelingt nach Standardmethoden der organischen Chemie durch Umsetzung von X mit Cyanidionen, z. B. mit Natrium- oder Kaliumcyanid (siehe EP-A 97460, Herstellungsbeispiel 1), Kupfer(I)-cyanid (siehe EP-A 34917, Herstellungsbeispiel 3) oder Trimethylsilylcyanid. Anschließend wird die so erhaltene Verbindung XI in Analogie zu den in Schema 1, Schritt ii) erläuterten Methoden in das Pyridin-N-oxid XII umgewandelt (Schritt x). Anschließend erfolgt in Schritt xi) die Umsetzung von XII mit einem Halogenierungsmittel wie POCl₃ oder POBr₃, wobei man die entsprechende Verbindung VII erhält. Zur Umsetzung von XII in Schritt ix) setzt man das Halogenierungsmittel in der Regel im Überschuss, bezogen auf die Stöchiometrie der Reaktion ein. Die Umsetzung kann in einem inerten organischen Lösungsmittel durchgeführt werden und erfolgt häufig in Abwesenheit eines Lösungsmittels, wobei dann in der Regel das Halogenierungsmittel als Lösungsmittel fungiert. Die Reaktionstemperatur liegt üblicherweise im Bereich von 20 °C bis zur Siedetemperatur des Halogenierungsmittels. Anschließend kann man die Verbindung VII nach der in Schema 2 dargestellten Methode in die Verbindung I umwandeln. Alternativ kann man zunächst die Verbindung VII mit Phenylboronsäure nach den in Schema 1, Schritt i) bzw. Schema 2, Schritt viii) genannten Methoden umsetzen, wobei man die Verbindung der allgemeinen Formel V erhält, die man dann gemäß den Schritten iv) und v) des Schemas 1 in die erfindungsgemäße Verbindung der allgemeinen Formel I umwandelt.

Anstelle der Boronsäure-(Derivate) IIa können in Schema 1 Schritt, i), Schema 2 Schritt viii) und Schema 3, Schritt xii) auch Phenylgrignard-Verbindungen der allgemeinen Formel IIb eingesetzt werden.

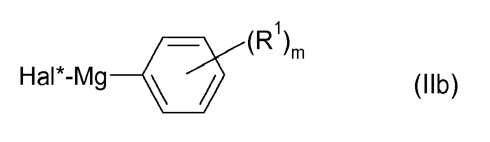

In Formel IIb haben R¹ und m die zuvor genannten Bedeutungen. Hal* steht für Chlor, Brom oder Iod. Die Kupplungsreaktion in Schema 1 Schritt, i), Schema 2 Schritt viii) und Schema 3, Schritt xii) erfolgt dann in Gegenwart der zuvor erwähnten Palladiumkatalysatoren oder in Gegenwart von Tris(acetylacetonato)eisen(III) (siehe Tetrahedron Lett. S. 3547 (2002)) unter gegebenenfalls geringfügig modifizierten Bedingungen, wobei üblicherweise der Katalysator in einer Menge von 0,2 bis 8 mol-%, insbesondere 0,5 bis 5 mol-%, bezogen auf die zu kuppelnde Grignard-Verbindung IIb eingesetzt wird. Besonders bevorzugter Katalysator ist [1,4-Bis(diphenylphosphin)-butan]palladium(II)chlorid . Die Umsetzungen erfolgen in der Regel bei Temperaturen im Bereich von -40 bis +120 °C und insbesondere im Bereich von 20 bis 100°C. Die Umsetzungen erfolgen üblicherweise in einem inerten, aprotischen organischen Lösungsmittel, vorzugsweise einem Ether und insbesondere einem cyclischen Ether wie Tetrahydrofuran oder in einer Mischung verschiedener aprotischer inerter Lösungsmittel, wobei vorzugsweise eines der Lösungsmittel ein cyclischer Ether wie Tetrahydrofuran ist. Beispiele für derartige Mischungen sind Tetrahydrofuran/N-Methylpyrrolidon, Tetrahydrofuran/Toluol oder Xylol, Tetrahydrofuran/Dioxan, Tetrahydrofuran/N,N-Dimethylpropylenharnstoff (DMPU) sowie Tetrahydrofuran/Sulfolan.

Ein weiterer Zugang zu den Verbindungen der allgemeinen Formel I, mit R² = H, Alkyl, Alkoxy oder insbesondere CN wird in Schema 4 erläutert.

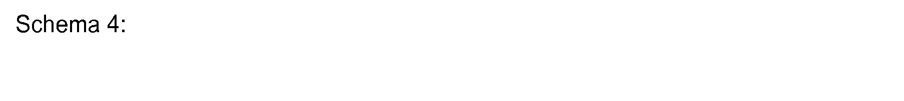

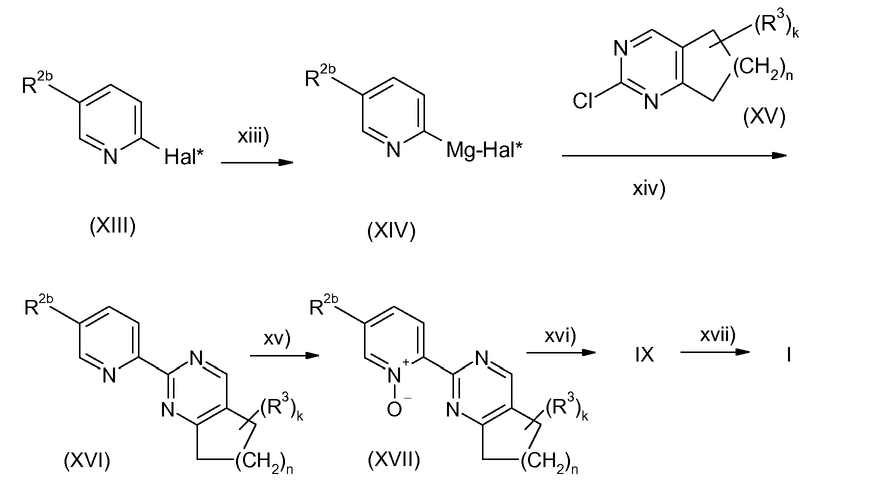

In Schema 4 haben n, R³ und k die zuvor genannten Bedeutungen. R^{2b} steht für H, C₁-C₄-Alkyl oder C₁-C₄-Alkoxy und insbesondere für CN. Hal* steht für Chlor, Brom oder insbesondere Iod. Die Herstellung der Grignard-Verbindung XIV aus dem 2-Halogenpyridin XIII kann nach an sich bekannten Verfahren erfolgen, wie sie beispielsweise in Synlett S. 1359 (1998) beschrieben sind.

Die anschließenden Kupplung von XIV mit der 2-Chlorpyrimidinverbindung XV in Schritt xiv) erfolgt analog zu der bereits erläuterten Kupplung von Grignard-Verbindungen IIb. Vorzugsweise erfolgt die Kupplung in Gegenwart eines Übergangsmetallkatalysators eines Metalls der Gruppe 8 bis 10 (nach IUPAC 1989), insbesondere eines Palladium-, Nickel oder Eisenkatalysators. Diesbezüglich wird auf die vorgenannten Katalysatoren Bezug genommen. Die Umsetzung erfolgt in einem hierfür üblichen Lösungsmittel, beispielsweise einem Ether, wie Diethylether, Dioxan, Tetrahydrofuran, einem aromatischen Kohlenwasserstoff wie Toluol oder Xylole, oder einem aprotischen Amid, Lactam oder Harnstoff wie N-Methylpyrrolidon oder Dimethylpropylenharnstoff oder in Gemischen dieser Lösungsmittel, insbesondere Gemischen, die wenigstens einen Ether enthalten. Die Reaktionstemperaturen liegen in der Regel im Bereich von -40 bis +120 °C und insbesondere im Bereich von 20 bis 100 °C. Wegen weiterer Details wird auf die in J. Am. Chem. Soc. 124, S. 13856 (2002), Chem. Pharm. Bull., S. 4533 (1983) und Chem. Pharm. Bull., S. 2005 (1984) beschriebenen Methoden verwiesen, die in analoger Weise zur Kupplung von XIV mit XV angewendet werden können.

Die so erhaltene Verbindung XVI wird dann in Schritt xv) in das N-Oxid XVII überführt. Bezüglich Schritt xv) wird auf das zu Schritt ii) in Schema 1 bzw. Schritt x) in Schema 3 Gesagte Bezug genommen. Anschließend wird in Schritt xvi) das N-Oxid XVII in Analogie zu Schritt xi in Schema 3 mit einem Halogenierungsmittel wie POCl₃ oder POBr₃ umgesetzt, wobei man die 2-Halogenverbindung IX aus Schema 2 erhält. Diese wird dann nach der in Schema 2 Schritt viii) angegebenen Methode mit einer Phenylboronsäureverbindung IIa oder der entsprechenden Grignard-Verbindung IIb umgesetzt, wobei man die Verbindung der allgemeinen Formel I verhält.

Verbindungen der allgemeinen Formel XV sind bekannt oder können nach an sich bekannten Methoden der organischen Chemie hergestellt werden (siehe beispielsweise US 6040448, WO 99/21850 und Chem. Pharm. Bull S. 2254 (1983)).

Die nach den in den Schemata 1 bis 4 dargestellten Methoden erhaltenen Reaktionsgemische werden in üblicher Weise aufgearbeitet, z. B. durch Mischen mit Wasser, Trennung der Phasen und gegebenenfalls chromatographische Reinigung der Rohprodukte. Die Zwischen- und Endprodukte fallen z. T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Sofern einzelne Verbindungen I nicht auf den voranstehend beschriebenen Wegen zugänglich sind, können sie durch Derivatisierung anderer Verbindungen I hergestellt werden.

Sofern bei der Synthese Isomerengemische anfallen, ist im allgemeinen jedoch eine Trennung nicht unbedingt erforderlich, da sich die einzelnen Isomere teilweise während der Aufbereitung für die Anwendung oder bei der Anwendung (z. B. unter Licht-, Säure- oder Baseneinwirkung) ineinander umwandeln können. Entsprechende Umwandlungen können auch nach der Anwendung, beispielsweise bei der Behandlung von Pflanzen in der behandelten Pflanze oder im zu bekämpfenden Schadpilz erfolgen.

Die Verbindungen I eignen sich als Fungizide. Sie zeichnen sich aus durch eine hervorragende Wirksamkeit gegen ein breites Spektrum von pflanzenpathogenen Pilzen, insbesondere aus der Klasse der *Ascomyceten, Deuteromyceten, Oomyceten* und *Basidiomyceten.* Sie sind zum Teil systemisch wirksam und können im Pflanzenschutz als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Bananen, Baumwolle, Soja, Kaffee, Zuckerrohr, Wein, Obst- und Zierpflanzen und Gemüsepflanzen wie Gurken, Bohnen, Tomaten, Kartoffeln und Kürbisgewächsen, sowie an den Samen dieser Pflanzen.

Speziell eignen sie sich zur Bekämpfung folgender Pflanzenkrankheiten:
- *Alternaria-Arten* an Gemüse und Obst,
- *Bipolaris-* und *Drechslera-Arten* an Getreide, Reis und Rasen,
- *Blumeria graminis* (echter Mehltau) an Getreide,
- *Botrytis cinerea* (Grauschimmel) an Erdbeeren, Gemüse, Zierpflanzen und Reben,
- *Erysiphe cichoracearum* und *Sphaerotheca fuliginea* an Kürbisgewächsen,
- *Fusarium-* und *Verticillium-Arten* an verschiedenen Pflanzen,
- *Mycosphaerella-Arten* an Getreide, Bananen und Erdnüssen,
- *Phytophthora infestans* an Kartoffeln und Tomaten,
- *Plasmopara viticola* an Reben,
- *Podosphaera leucotricha* an Äpfeln,
- *Pseudocercosporella herpotrichoides* an Weizen und Gerste,
- *Pseudoperonospora-Arten* an Hopfen und Gurken,
- *Puccinia-Arten* an Getreide,
- *Pyricularia oryzae* an Reis,
- *Rhizoctonia-Arten* an Baumwolle, Reis und Rasen,
- *Septoria tritici* und *Stagonospora nodorum* an Weizen,
- *Uncinula necator* an Reben,
- *Ustilago*-Arten an Getreide und Zuckerrohr, sowie
- *Venturia-Arten* (Schorf) an Äpfeln und Birnen.

Die Verbindungen I eignen sich außerdem zur Bekämpfung von Schadpilzen wie *Paecilomyces variotii* im Materialschutz (z. B. Holz, Papier, Dispersionen für den Anstrich, Fasern bzw. Gewebe) und im Vorratsschutz.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung kann sowohl vor als auch nach der Infektion der Materialien, Pflanzen oder Samen durch die Pilze erfolgen.

Die fungiziden Mittel enthalten im allgemeinen zwischen 0,1 und 95, vorzugsweise zwischen 0,5 und 90 Gew.-% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Kubikmeter behandelten Materials.

Die Verbindungen I können in die üblichen Formulierungen überführt werden, z. B. Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten.

Die Formulierungen werden in bekannter Weise hergestellt, z. B. durch Verstrecken des Wirkstoffs mit Lösungsmitteln und/oder Trägerstoffen, gewünschtenfalls unter Verwendung von Emulgiermitteln und Dispergiermitteln. Als Lösungsmittel / Hilfsstoffe kommen dafür im wesentlichen in Betracht:
- Wasser, aromatische Lösungsmittel (z. B. Solvesso Produkte, Xylol), Paraffine (z. B. Erdölfraktionen), Alkohole (z. B. Methanol, Butanol, Pentanol, Benzylalkohol), Ketone (z. B. Cyclohexanon, gamma-Butryolacton), Pyrrolidone (NMP, NOP), Acetate (Glykoldiacetat), Glykole, Dimethylfettsäureamide, Fettsäuren und Fettsäureester. Grundsätzlich können auch Lösungsmittelgemische verwendet werden,
- Trägerstoffe wie natürliche Gesteinsmehle (z. B. Kaoline, Tonerden, Talkum, Kreide) und synthetische Gesteinsmehle (z. B. hochdisperse Kieselsäure, Silikate); Emulgiermittel wie nichtionogene und anionische Emulgatoren (z. B. Polyoxyethylen-Fettalkohol-Ether, Alkylsulfonate und Arylsulfonate) und Dispergiermittel wie Lignin-Sulfitablaugen und Methylcellulose.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate, Fettsäuren und sulfatierte Fettalkoholglykolether zum Einsatz, ferner Kondensationsprodukte von sulfoniertem Naphthalin und Naphthalinderivaten mit Formaldehyd, Kondensationsprodukte des Naphthalins bzw. der Naphthalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Tristearylphenylpolyglykolether, Alkyl-arylpolyetheralkohole, Alkohol- und Fettalkoholethylenoxid-Kondensate, ethoxyliertes Rizinusöl, Polyoxyethylenalkylether, ethoxyliertes Polyoxypropylen,
Laurylalkoholpolyglykoletheracetal, Sorbitester, Ligninsulfitablaugen und Methylcellulose in Betracht.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z. B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Isophoron, stark polare Lösungsmittel, z. B. Dimethylsulfoxid, N-Methylpyrrolidon oder Wasser in Betracht.

Pulver-, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an feste Trägerstoffe hergestellt werden. Feste Trägerstoffe sind z. B. Mineralerden, wie Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehl, Baumrinden-, Holz- und Nussschalenmehl, Cellulosepulver und andere feste Trägerstoffe.

Die Formulierungen enthalten im allgemeinen zwischen 0,01 und 95 Gew.-%, vorzugsweise zwischen 0,1 und 90 Gew.-% des Wirkstoffs. Die Wirkstoffe werden dabei in einer Reinheit von 90 % bis 100 %, vorzugsweise 95 % bis 100 % (nach NMR-Spektrum) eingesetzt.

Beispiele für Formulierungen umfassen Produkte zur Verdünnung in Wasser, z. B.
- A: Wasserlösliche Konzentrate (SL):
10 Gew.-Teile einer erfindungsgemäßen Verbindung werden in Wasser oder einem wasserlöslichen Lösungsmittel gelöst. Alternativ werden Netzmittel oder andere Hilfsmittel zugefügt. Bei der Verdünnung in Wasser löst sich der Wirkstoff;
- B: Dispergierbare Konzentrate (DC):
20 Gew.-Teile einer erfindungsgemäßen Verbindung werden in Cyclohexanon unter Zusatz eines Dispergiermittels z. B. Polyvinylpyrrolidon gelöst. Bei Verdünnung in Wasser ergibt sich eine Dispersion;
- C: Emulgierbare Konzentrate (EC):
15 Gew.-Teile einer erfindungsgemäßen Verbindung werden in Xylol unter Zusatz von Ca-Dodecylbenzolsulfonat und Ricinusölethoxylat (jeweils 5 %) gelöst. Bei der Verdünnung in Wasser ergibt sich eine Emulsion;
- D: Emulsionen (EW, EO):
40 Gew.-Teile einer erfindungsgemäßen Verbindung werden in Xylol unter Zusatz von Ca-Dodecylbenzolsulfonat und Ricinusölethoxylat (jeweils 5 %) gelöst. Diese Mischung wird mittels einer Emulgiermaschine (Ultraturax) in Wasser eingebracht und zu einer homogenen Emulsion gebracht. Bei der Verdünnung in Wasser ergibt sich eine Emulsion,
- E: Suspensionen (SC, OD):
20 Gew.-Teile einer erfindungsgemäßen Verbindung werden unter Zusatz von Dispergier- und Netzmitteln und Wasser oder einem organischen Lösungsmittel in einer Rührwerkskugelmühle zu einer feinen Wirkstoffsuspension zerkleinert. Bei der Verdünnung in Wasser ergibt sich eine stabile Suspension des Wirkstoffs;
- F: Wasserdispergierbare und wasserlösliche Granulate (WG, SG):
50 Gew.-Teile einer erfindungsgemäßen Verbindung werden unter Zusatz von Dispergier- und Netzmitteln fein gemahlen und mittels technischer Geräte (z. B. Extrusion, Sprühturm, Wirbelschicht) als wasserdispergierbare oder wasserlösliche Granulate hergestellt. Bei der Verdünnung in Wasser ergibt sich eine stabile Dispersion oder Lösung des Wirkstoffs;
- G: Wasserdispergierbare und wasserlösliche Pulver (WP, SP):
75 Gew.-Teile einer erfindungsgemäßen Verbindung werden unter Zusatz von Dispergier- und Netzmitteln sowie Kieselsäuregel in einer Rotor-Strator Mühle vermahlen. Bei der Verdünnung in Wasser ergibt sich eine stabile Dispersion oder Lösung des Wirkstoffs;
sowie Produkte für die Direktapplikation, z. B.
- H: Stäube (DP):
5 Gew.-Teile einer erfindungsgemäßen Verbindung werden fein gemahlen und mit 95 % feinteiligem Kaolin innig vermischt. Man erhält dadurch ein Stäubemittel;
- I: Granulate (GR, FG, GG, MG):
0.5 Gew.-Teile einer erfindungsgemäßen Verbindung werden fein gemahlen und mit 95,5 % Trägerstoffe verbunden. Gängige Verfahren sind dabei die Extrusion, die Sprühtrocknung oder die Wirbelschicht. Man erhält dadurch ein Granulat für die Direktapplikation;
- J: ULV- Lösungen (UL);
10 Gew.-Teile einer erfindungsgemäßen Verbindung werden in einem organischen Lösungsmittel z. B. Xylol gelöst. Dadurch erhält man ein Produkt für die Direktapplikation.

Die Wirkstoffe können als solche, in Form ihrer Formulierungen oder den daraus bereiteten Anwendungsformen, z. B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Wässrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Die Wirkstoffkonzentrationen in den anwendungsfertigen Zubereitungen können in größeren Bereichen variiert werden. Im Allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Zu den Wirkstoffen können Öle verschiedenen Typs, Netzmittel, Adjuvants, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Die erfindungsgemäßen Mittel können in der Anwendungsform als Fungizide auch zusammen mit anderen Wirkstoffen vorliegen, der z. B. mit Herbiziden, Insektiziden, Wachstumsregulatoren, Fungiziden oder auch mit Düngemitteln. Beim Vermischen der Verbindungen I bzw. der sie enthaltenden Mittel in der Anwendungsform als Fungizide mit anderen Fungiziden erhält man in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
- Acylalanine wie Benalaxyl, Metalaxyl, Ofurace, Oxadixyl,
- Aminderivate wie Aldimorph, Dodine, Dodemorph, Fenpropimorph, Fenpropidin, Guazatine, Iminoctadine, Spiroxamin, Tridemorph
- Anilinopyrimidine wie Pyrimethanil, Mepanipyrim oder Cyprodinyl,
- Antibiotika wie Cycloheximid, Griseofulvin, Kasugamycin, Natamycin, Polyoxin oder Streptomycin,
- Azole wie Bitertanol, Bromoconazol, Cyproconazol, Difenoconazole, Dinitroconazol, Epoxiconazol, Fenbuconazol, Fluquinconazol, Flusilazol, Hexaconazol, Imazalil, Metconazol, Myclobutanil, Penconazol, Propiconazol, Prochloraz, Prothioconazol, Tebuconazol, Triadimefon, Triadimenol, Triflumizol, Triticonazol,
- Dicarboximide wie Iprodion, Myclozolin, Procymidon, Vinclozolin,
- Dithiocarbamate wie Ferbam, Nabam, Maneb, Mancozeb, Metam, Metiram, Propineb, Polycarbamat, Thiram, Ziram, Zineb,
- Heterocylische Verbindungen wie Anilazin, Benomyl, Boscalid, Carbendazim, Carboxin, Oxycarboxin, Cyazofamid, Dazomet, Dithianon, Famoxadon, Fenamidon, Fenarimol, Fuberidazol, Flutolanil, Furametpyr, Isoprothiolan, Mepronil, Nuarimol, Probenazol, Proquinazid, Pyrifenox, Pyroquilon, Quinoxyfen, Silthiofam, Thiabendazol, Thifluzamid, Thiophanat-methyl, Tiadinil, Tricyclazol, Triforine,
- Kupferfungizide wie Bordeaux Brühe, Kupferacetat, Kupferoxychlorid, basisches Kupfersulfat,
- Nitrophenylderivate, wie Binapacryl, Dinocap, Dinobuton, Nitrophthal-isopropyl
- Phenylpyrrole wie Fenpiclonil oder Fludioxonil,
- Schwefel
- Sonstige Fungizide wie Acibenzolar-S-methyl, Benthiavalicarb, Carpropamid, Chlorothalonil, Cyflufenamid, Cymoxanil, Diclomezin, Diclocymet, Diethofencarb, Edifenphos, Ethaboxam, Fenhexamid, Fentin-Acetat, Fenoxanil, Ferimzone, Fluazinam, Fosetyl, Fosetyl-Aluminium, Iprovalicarb, Hexachlorbenzol, Metrafenon, Pencycuron, Propamocarb, Phthalid, Toloclofos-methyl, Quintozene, Zoxamid
- Strobilurine wie Azoxystrobin, Dimoxystrobin, Fluoxastrobin, Kresoxim-methyl, Metominostrobin, Orysastrobin, Picoxystrobin, Pyraclostrobin oder Trifloxystrobin,
- Sulfensäurederivate wie Captafol, Captan, Dichlofluanid, Folpet, Tolylfluanid
- Zimtsäureamide und Analoge wie Dimethomorph, Flumetover oder Flumorph.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen benutzt. Die so erhaltenen Verbindungen sind in den anschließenden Tabellen mit physikalischen Angaben aufgeführt.

In den Beispielen werden die folgenden Abkürzungen verwendet:
Fp.: Festpunkt;
MtBE: Methyl-tert.-butylether;
EtOH: Ethanol.
In Verbindung mit den ¹H-NMR-Daten werden die folgenden Abkürzungen benutzt:
s: Singulett; d: Dublett; t: triplett; m: multiplett

### Beispiel 1: 2-(5-Methyl-6-phenylpyridin-2-yl)-6,7,8,9-tetrahydro-5H-cycloheptapyrimidin

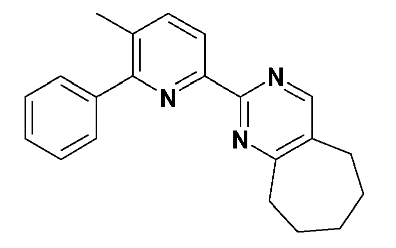

### 1.1 3-Methyl-2-phenylpyridin

Zu einer Lösung von 2,0 g (11,6 mmol) 2-Brom-3-methylpyridin in 80 ml Tetrahydrofuran gab man nacheinander 2,1 g (17,2 mmol) Phenylboronsäure und 4,3 g Kaliumcarbonat in 20 ml Wasser. Nach Zugabe von 300 mg Tetrakis-(triphenylphosphin)palladium(0) rührte man 8 Stunden unter Rückfluss. Die Reaktionslösung wurde auf Eiswasser gegeben und mit MtBE extrahiert. Die vereinigten organischen Phasen wurden getrocknet, das Lösungsmittel im Vakuum entfernt und der Rückstand über Kieselgel mit Cyclohexan/MtBE (9:1) chromatographiert. Man erhielt so 0,8 g Produkt.
¹H-NMR (δ,CDCl₃,): 2,5 (s); 7,2 (m); 7,35-7,6 (m) und 8,5 (m)

### 1.2 3-Methyl-2-phenylpyridin-N-oxid

25 g (147,7 mmol) 3-Methyl-2-phenylpyridin wurden in 150 ml Dichlormethan vorgelegt. Bei 5 °C gab man portionsweise 51,6 g (294 mmol) 3-Chlorperoxybenzoesäure zu und ließ 2 Stunden bei 5 °C sowie 18 Stunden bei 23 °C nachrühren. Nach Entfernen des Lösungsmittels wurde der Rückstand über Kieselgel mit Cyclohexan/MtBE (1:1) chromatographiert und man erhielt 25 g Produkt.
Fp.: 163-165 °C.
¹H-NMR (δ, CDCl₃,)_{:} 2,0 (s); 7,1-7,6 (m); 8,3 (m).

### 1.3 5-Methyl-6-phenylpyridin-2-carbonnitril

Zu einer Lösung von 25 g (118,2 mmol) der im Beispiel 1.2 hergestellten Verbindung in 150 ml Dichlormethan gab man 14,9 (150 mmol) Trimethylsilylcyanid und rührte 30 Minuten bei Raumtemperatur nach. Anschließend gab man eine Lösung von 16,2 g (150 mmol) Dimethylcarbamoylchlorid innerhalb 45 min zu und ließ 18 Stunden bei 23 °C nachrühren. Zu der Reaktionslösung gab man vorsichtig 70 ml Wasser und 40 ml 1 N Natronlauge. Anschließend wurde mit festem Natriumcarbonat der pH-Wert auf 8 eingestellt. Die organische Phase wurde abgetrennt, mit Wasser gewaschen und getrocknet. Nach Entfernen des Lösungsmittels erhielt man nach Chromatographie über Kieselgel mit Cyclohexan/MtBE (3:2) 19,9 g der Titelverbindung als Öl.
¹H-NMR (δ,CDCl₃,): 2,45 (s) ; 7,4-7,8 (m)

### 1.4 5-Methyl-6-phenyl-pyridin-2-carboxamidin-Hydrochlorid

Zu einer Lösung von 5,0 g (26 mmol) 5-Methyl-6-phenylpyridin-2-carbonnitril aus Beispiel 1.3 in 65 ml Methanol gab man 2,34 g einer 30%igen Natriummethylat-Lösung in Methanol und rührte 7 Stunden bei 23 °C nach. Anschließend gab man 1,5 g Ammoniumchlorid zu und ließ weitere 8 Stunden bei 23 °C nachrühren. Nach Entfernen des Lösungsmittels gab man MtBE zu und filtrierte das Produkt ab, wobei man 5,4 g der Titelverbindung als gelblichen Feststoff erhielt.
¹H-NMR (δ, DMSO): 7,5 (m); 7,7 (m); 8,2 (m); 8,3 (m); 9,6 (m).

### 1.5 2-(5-Methyl-6-phenyl-pyridin-2-yl)-6,7,8,9-tetrahydro-5H-cycloheptapyrimidin

Zu einer Lösung von 1,5 g (6,1 mmol) der im Beispiel 1.4 hergestellten Verbindung in 30 ml Methanol gab man 1,3 g Natriummethylat (30%ige Lösung in Methanol). Nach 30 min gab man hierzu 1,2 g (7,3 mmol) 2-Dimethylaminomethylen-cycloheptanon [hergestellt nach Tetrahedron Letters 2567 (1986)] und erhitzte die Mischung 2 Stunden zum Rückfluss. Die Reaktionslösung wurde danach zwischen Wasser und MtBE verteilt. Die organische Phase wurde abgetrennt. Das Lösungsmittel wurde unter vermindertem Druck entfernt und der Rückstand wurde über Kieselgel mit Cyclohexan/MtBE (1:1) chromatographiert. Man erhielt so 0,72 g der Titelverbindung.
Fp.: 151-154 °C

### Beispiel 2: 4-[3-Methyl-6-(5,6,7,8-tetrahydrochinazolin-2-yl)-pyridin-2-yl]-benzaldehyd

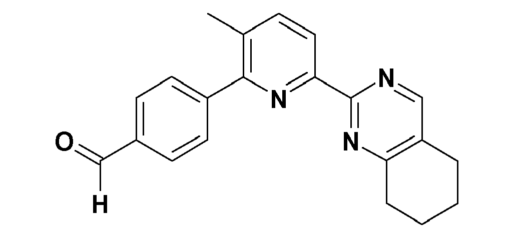

### 2.1 6-Brom-5-methylpyridin-2-carboxamidin-Hydrochlorid

Zu 4,90 g (25 mmol) 6-Brom-5-methylpyridin-2-carbonitril [hergestellt nach US 2003/0087940 A1 bzw. Bioorg. Med. Chem. Lett. 1571-1574 (2003)] in 60 ml Methanol gab man 2,2 g einer 30%igen Natriummethylat-Lösung in Methanol und rührte 7 Stunden bei 23 °C nach. Anschließend gab man 1,5 g Ammoniumchlorid zu und ließ weitere 8 Stunden bei 23 °C nachrühren. Nach Entfernen des Lösungsmittels gab man zu dem Rückstand MtBE und filtrierte den Feststoff ab. Man erhielt so 4,2 g der Titelverbindung als weißen Feststoff der ohne Aufreinigung weiter umgesetzt wurde.

### 2.2 2-(6-Brom-5-methylpyridin-2-yl)-5,6,7,8-tetrahydrochinazolin

Zu einer Lösung von 4,2 g (7 mmol) der im Beispiel 2.1 hergestellten Verbindung in 100 ml Methanol gab man 3,6 g Natriummethylat (30%ige Lösung in Methanol). Nach 30 min gab man hierzu 3,1 g (20 mmol) 2-Dimethylaminomethylencyclohexanon [hergestellt z.B. nach Tetrahedron 50(7), 2255-64 (1994); Synthetic Communications 28(10), 1743-1753 (1998) oder Tetrahedron Letters 27(23), 2567-70 (1986)] und erhitzte die Mischung 2 Stunden zum Rückfluss. Die Reaktionslösung wurde danach zwischen Wasser und MtBE verteilt. Die organische Phase wurde abgetrennt, das Lösungsmittel wurde im Vakuum entfernt und der Rückstand wurde über Kieselgel mit Cyclohexan/MtBE (1:1) chromatographiert. Man erhielt so 2,2 g der Titelverbindung.
¹H-NMR (δ,CDCl₃,): 1,8-2,0 (m); 2,5 (s); 2,8 (m); 3,0 (m)

### 2.3 4-[3-Methyl-6-(5,6,7,8-tetrahydrochinazolin-2-yl)-pyridin-2-yl]-benzaldehyd

Zu einer Lösung von 0,2 g der im Beispiel 2.2 hergestellten Verbindung in 20 ml Ethylenglykoldimethylether gab man nacheinander 0,24 g 4-Formylphenylboronsäure und 0,2 g Natriumcarbonat in 3 ml Wasser. Nach Zugabe von ca. 50 mg Tetrakis(triphenylphosphin)palladium(0) rührte man 9 Stunden unter Rückfluss. Anschließend gab man weitere 0,2 g 4-Formyl-phenylboronsäure zu und ließ noch 10 Stunden unter Rückfluss reagieren. Die Reaktionslösung wurde danach zwischen Wasser und MtBE verteilt. Die organische Phase wurde abgetrennt, das Lösungsmittel wurde im Vakuum entfernt und der Rückstand wurde über Kieselgel mit Cyclohexan/MtBE (1:1) chromatographiert. Man erhielt so 35 mg der Titelverbindung mit einem Fp. von 151-154°C.
¹H-NMR (δ,CDCl₃,): 1,8-2,0 (m, 4 H); 2,4 (s, 3H); 2,8 (m, 2H); 3,0 (m, 2H); 7,8 (m, 3H); 8,0 (m, 2H); 8,4 (m, 1H); 8,6 (s, 1H); 10,1 (s, 1H).

### Beispiel 3: 2-(5-Methoxy-6-phenylpyridin-2-yl)-5,6,7,8-tetrahydrochinazolin

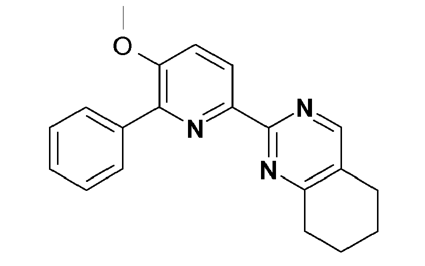

### 3.1 5-Methoxy-6-phenyl-pyridin-2-carbonitril

2,1 g 3-Methoxy-2-phenylpyridin [hergestellt nach Bulletin de la Societe Chimique de France 1112-16 (1974)] wurden in 60 ml Dichlormethan vorgelegt. Bei 5 °C versetzte man portionsweise mit 2,4 g 3-Chlorperoxybenzoesäure und ließ 2 Stunden bei 5 °C sowie 18 Stunden bei 23 °C nachrühren. Nach Entfernen von Lösungsmittel wurde der Rückstand über Kieselgel mit Methyl-tert.-butylether (MtBE) chromatographiert und man erhielt 1,4 g 3-Methoxy-2-phenylpyridin-N-oxid als Rohprodukt in Form eines Öls.

Das Rohprodukt löste man in 80 ml Dichlormethan und tropfte hierzu innerhalb 5 min 0,9 g Trimethylsilylcyanid und rührte 30 Minuten bei 23 °C nach. Anschließend tropfte man innerhalb 45 min eine Lösung von 0,95 g Dimethylcarbamoylchlorid in 10 ml Dichlormethan zu und ließ 18 Stunden bei 23 °C nachrühren. Die Reaktionslösung versetzte man vorsichtig mit 40 ml Wasser und 10 ml 1 N Natronlauge. Anschließend stellte man mit festem Natriumcarbonat den pH-Wert auf 8 ein, trennte die organische Phase ab, wusch mit Wasser und trocknete. Nach Entfernen des Lösungsmittels erhielt man nach Chromatographie über Kieselgel mit Cyclohexan/MtBE (3:2) 0,2 g 5-Methoxy-6-phenylpyridin-2-carbonitril als Öl.
¹H-NMR (δ,CDCl₃,): 3,9 (s); 7,5 (m); 7,7 (m) und 7,9 (m).

### 3.2 2-(5-Methoxy-6-phenylpyridin-2-yl)-5,6,7,8-tetrahydrochinazolin

Die Titelverbindung kann in Analogie zu den Schritten 1.4 und 1.5 des Beispiels 1 hergestellt werden, wobei man anstelle von 2-Dimethylaminomethylencycloheptanon das entsprechende Hexanon einsetzt.

### Beispiel 4: 2-(5-Chlor-6-phenylpyridin-2-yl)-5,6,7,8-tetrahydrochinazolin

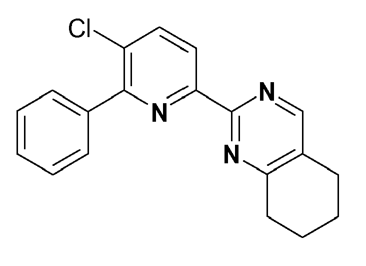

### 4.1 2-Brom-2-chlor-pyridin

6,3 g 2,3-Dichlorpyridin in 50 ml Essigsäure versetzte man mit 60 ml einer 33%igen Bromwasserstoff-Lösung in Essigsäure und erhitzte 8 Stunden zum Rückfluss. Anschließend gab man weitere 42 ml Bromwasserstoff-Lösung zu. Nach 6 Stunden war die Umsetzung vollständig. Die Reaktionslösung wurde auf Eiswasser gegeben und mit Methylenchlorid extrahiert. Die vereinigten organischen Phasen wurden mit Wasser gewaschen, getrocknet und das Lösungsmittel im Vakuum entfernt. So erhielt man 8,7 g Produkt als Öl.
¹H-NMR (δ, CDCl₃,): 7,2 (m); 7,8 (m) und 8,3 (m).

### 4.2 3-Chlor-2-phenylpyridin

Eine Lösung von 2,5 g 2-Brom-3-methoxypyridin in 80 ml Tetrahydrofuran versetzte man nacheinander mit 2,35 g Phenylboronsäure und 4,8 g Natrimcarbonat in 30 ml Wasser. Nach Zugabe von 300 mg Tetrakis-(triphenylphosphin)-palladium(0) rührte man 8 Stunden unter Rückfluss. Anschließend gab man noch 2 g Phenylboronsäure, 2 g Natriumcarbonat sowie 100 mg Tetrakis-(triphenylphosphin)palladium(0) und erhitzte weitere 6 Stunden unter Rückfluss. Die Reaktionslösung wurde auf Eiswasser gegeben und mit MtBE extrahiert. Die vereinigten organischen Phasen wurden getrocknet, das Lösungsmittel im Vakuum entfernt und der Rückstand über Kieselgel mit Cyclohexan/MtBE (9:1) chromatographiert. Man erhielt so 2,2 g Produkt als Öl.
¹H-NMR (δ, CDCl₃,): 7,3 (m); 7,5 (m); 7,8 (m) und 8,6 (m).

### 4.3 3-Chlor-2-phenyl-pyridin-1-oxid

2,2 g 3-Chlor-2-phenylpyridin wurden in 80 ml Dichlormethan vorgelegt. Bei 5°C versetzte man die Mischung portionsweise mit 3,0 g 3-Chlorperoxybenzoesäure und ließ 2 Stunden bei 5 °C sowie 18 Stunden bei 23 °C nachrühren. Nach Entfernen von Lösungsmittel wurde der Rückstand über Kieselgel mit MtBE chromatographiert und man erhielt 1,9 g Produkt als Öl.
¹H-NMR (δ,CDCl₃,): 7,2 (m); 7,4-7,6 (m) und 8,3 (m).

### 4.4 5-Chlor-6-phenyl-pyridin-2-carbonitril

1,2 g 3-Chlor-2-phenyl-pyridin-1-oxid in 5 ml DMF versetzte man mit 0,74 g Dimethylsulfat und ließ 7 Stunden bei 60 °C reagieren. Nach Abkühlen auf 23 °C wurde diese Lösung zu 0,38 g Kaliumcyanid in 10 ml DMF getropft und 18 Stunden bei 23 °C nachgerührt. Anschließend verteilte man zwischen MtBE und Wasser, die organische Phase wurden getrocknet, das Lösungsmittel im Vakuum entfernt und der Rückstand über Kieselgel mit Cyclohexan/MtBE (3:2) chromatographiert. Man erhielt so 0,30 g Produkt.
¹H-NMR (δ, CDCl₃,): 7,5 (m); 7,6 (m); 7,7 (m) und 7,9 (m).

### 4.5 2-(5-Chlor-6-phenylpyridin-2-yl)-5,6,7,8-tetrahydrochinazolin

Die Titelverbindung kann in Analogie zu den Schritten 1.4 und 1.5 des Beispiels 1 hergestellt werden, wobei man anstelle von 2-Dimethylaminomethylencycloheptanon das entsprechende Hexanon einsetzt.

### Beispiel 5: 4-[3-Methyl-6-(5,6,7,8-tetrahydrochinazolin-2-yl)-pyridin-2-yl]-benzonitril

### 5.1 6-Chlor-5-methylpyridin-2-carbonitril

2,3 g 5-Methylpyridin-2-carbonitril wurden in 80 ml Dichlormethan vorgelegt. Bei 5 °C gab man hierzu portionsweise mit 5,4 g 3-Chlorperoxybenzoesäure und ließ 2 Stunden bei 5 °C sowie 18 Stunden bei 23 °C nachrühren. Nach Entfernen von Lösungsmittel wurde der Rückstand über Kieselgel mit MtBE chromatographiert und man erhielt 0,8 g 2-Cyano-5-methyl-pyridin-1-oxid.

0,8 g 2-Cyano-5-methyl-pyridin-1-oxid erhitzte man zusammen mit 25 ml Phosphoroxychlorid 5 Stunden unter Rückfluss. Nach vollständiger Reaktion entfernte man das überschüssige Phosphoroxychlorid im Vakuum. Der Rückstand wurde in Methylenchlorid aufgenommen, unter Eiskühlung auf Wasser gegeben und mit 3 N Natronlauge auf pH 12 gebracht. Die organische Phase wurde abgetrennt, getrocknet und das Lösungsmittel im Vakuum entfernt. So erhielt man 0,8 g Produkt.
¹H-NMR (δ,CDCl₃,): 2,4 (s); 7,6 (m) und 7,7 (m).

### 5.2 2-(6-Chlor-5-methyl-pyridin-2-yl)-5,6,7,8-tetrahydrochinazolin

Analog Beispiel 1.4 wurde 6-Chlor-5-methyl-pyridin-2-carbonitril in das 6-Brom-5-methylpyridin-2-carboxamidin-Hydrochlorid überführt. Anschließend wurde hieraus analog Beispiel 2.2 2-(6-Chlor-5-methyl-pyridin-2-yl)-5,6,7,8-tetrahydro-chinazolin hergestellt.
¹H-NMR (δ,CDCl₃,): 8,5 (s); 8,3 (m); 7,7 (m); 3,0 (m); 2,8 (m); 2,4 (s); 1,8-2,0 (m).

### 5.3 2-(6-Brom-5-methyl-pyridin-2-yl)-5,6,7,8-tetrahydrochinazolin

1 g 2-(6-Chlor-5-methyl-pyridin-2-yl)-5,6,7,8-tetrahydrochinazolin in 8 ml Essigsäure versetzte man mit 8 ml einer 33%igen Bromwasserstoff-Lösung in Essigsäure und erhitzte 10 Stunden unter Rückfluss. Die Reaktionslösung wurde mit Wasser verdünnt, mit 3 N Natronlauge auf pH 9 gebracht und mit MtBE extrahiert. Die vereinigten organischen Phasen wurden mit Wasser gewaschen, getrocknet und das Lösungsmittel im Vakuum entfernt. So erhielt man 0,9 g Produkt.
Fp.: 125-128 °C

### 5.4 4-[3-Methyl-6-(5,6,7,8-tetrahydrochinazolin-2-yl)-pyridin-2-yl]-benzonitril

Die Herstellung der Titelverbindung erfolgte analog Beispiel 2.3 durch Umsetzung von 2-(6-Brom-5-methyl-pyridin-2-yl)-5,6,7,8-tetrahydrochinazolin mit 4-Cyanophenylboronsäure.
Fp.: 178-182°C

### Herstellungsbeispiele für Ausgangsmaterialien:

### Herstellungsbeispiel 1: 2-(4-Fluorphenyl)-3-methyl-pyridin

Eine Lösung von 20,0 g 2-Brom-3-methylpyridin in 200 ml Tetrahydrofuran versetzte man mit 0,70 g [1,4 -Bis-(diphenylphosphino)-butan]-palladium(II)-chlorid. Nach 10 min tropfte man hierzu 128 ml einer 2 molaren Lösung von 4-Fluorphenylmagnesiumbromid in Tetrahydrofuran und erhitzte 5 Stunden unter Rückfluss. Nach Zugabe weiterer 30 ml 4-Fluorphenylmagnesiumbromid-Lösung wurde nach einer Stunde die Reaktionslösung unter Eiskühlung auf wässriger Ammoniumchlorid-Lösung gegeben und mit Essigester extrahiert. Die vereinigten organischen Phasen wurden getrocknet, das Lösungsmittel wurde im Vakuum entfernt und der Rückstand wurde über Kieselgel mit Cyclohexan/MtBE (9:1) chromatographiert. Man erhielt so 17,8 g 2-(4-Fluorphenyl)-3-methyl-pyridin als Öl.

Herstellungsbeispiel 2: 2-(6-Chlor-5-methyl-pyridin-2-yl)-5,6,7,8-tetrahydrochinazolin

### a) 5-Methyl-pyridin-2-carboxamidin-hydrochlorid

Die Verbindung wurde ausgehend von 2-Cyano-5-methylpyridin unter den für Beispiel 1.4 angegebenen Bedingungen hergestellt.
¹H-NMR (δ,DMSO): 2,4 (m); 7,9 (m); 8,3 (m); 8,6 (m).

### b) 2-(5-Methyl-pyridin-2-yl)-5,6,7,8-tetrahydro-quinazolin

Die Verbindung wurde ausgehend von 2-Methyl-pyridin-2-carboxamidin-hydrochlorid unter den für Beispiel 2.2 angegebenen Bedingungen hergestellt.
¹H-NMR (δ,CDCl₃,): 1,9 (m); 2,4 (s); 2,7 (m); 3,0 (m); 7,6 (m); 8,4 (m); 8,5 (m); 8,7 (m).

### c) 2-(5-Methyl-1-oxy-pyridin-2-yl)-5,6,7,8-tetrahydrochinazolin

1,0 g 2-(5-Methyl-pyridin-2-yl)-5,6,7,8-tetrahydrochinazolin wurden in 10 ml Dichlormethan vorgelegt. Bei 5 °C versetzte man portionsweise mit 1,4 g 3-Chlorperoxybenzoesäure und ließ 2 Stunden bei 5 °C sowie 18 Stunden bei 23 °C nachrühren. Nach Entfernen von Lösungsmittel wurde der Rückstand über Kieselgel mit MtBE/EtOH (5:2) chromatographiert und man erhielt 0,75 g Produkt.
¹H-NMR (δ,CDCl₃,): 8,6 (s); 8,2 (s); 7,5 (m); 7,1 (m); 3,0 (m); 2,8 (m); 2,4 (m); 1,7-1,9 (m).

### d) 2-(6-Chlor-5-methyl-pyridin-2-yl)-5,6,7,8-tetrahydrochinazolin

0,75 g 2-(5-Methyl-pyridin-2-yl)-5,6,7,8-tetrahydrochinazolin wurden zusammen mit 10 ml Phosphoroxychlorid 10 Stunden unter Rückfluß erhitzt. Nach vollständiger Reaktion entfernte man das überschüssige Phosphoroxychlorid im Vakuum. Der Rückstand wurde in Methylenchlorid aufgenommen und unter Eiskühlung auf Wasser gegeben. Die organische Phase wurde abgetrennt, getrocknet und das Lösungsmittel im Vakuum entfernt. Der Rückstand wurde über Kieselgel mit Cyclohexan/MtBE (4:1) chromatographiert. Man erhielt so 80 mg Produkt.

Nach den hier angegebenen Vorschriften wurden die in der nachfolgenden Tabelle B angegebenen Verbindungen der allgemeinen Formel I hergestellt.

**Tabelle B:**

| Beispiel | (R¹)ₘ * | R² | n | (R³)ₖ * | Phys. Daten (Fp. [°C]; ¹H-NMR ö [ppm]; MS m/e [M+H⁺]) |
|---|---|---|---|---|---|
| 1 | H | H | 3 | H | m/e 302 |
| 2 | 4-CHO | Methyl | 2 | H | 151-154 |
| 3 | H | OCH₃ | 2 | H | 138-141 |
| 4 | H | Cl | 2 | H | 133-136 |
| 5 | 4-CN | Methyl | 2 | H | 178-182 |
| 6 | H | Methyl | 3 | H | 150-153 |
| 7 | 4-Fluor | H | 3 | H | 90-93 |
| 8 | 4-Chlor | Methyl | 3 | H | 178-181 |
| 9 | 4-Fluor | Methyl | 3 | H | 163-165 |
| 10 | H | Methyl | 2 | 7,7-Dimethyl | 180-183 |
| 11 | H | H | 4 | H | 81-84 |
| 12 | H | Methyl | 4 | H | 152-158 |
| 13 | H | OCH₃ | 1 | H | 140-143 |
| 14 | H | OCH₃ | 3 | H | 156-159 |
| 15 | H | OCH₃ | 4 | H | |
| 16 | 4-F | OCH₃ | 1 | H | 148-150 |
| 17 | 4-F | OCH₃ | 2 | H | 156-158 |
| 18 | 4-F | OCH₃ | 3 | H | 162-164 |
| 19 | 4-Cl | OCH₃ | 1 | H | |
| 20 | 4-Cl | OCH₃ | 2 | H | |
| 21 | 4-Cl | OCH₃ | 3 | H | 136-139 |
| 22 | H | Cl | 1 | H | |
| 23 | H | Cl | 3 | H | 161-164 |
| 24 | H | Cl | 4 | H | |
| 25 | 4-F | Cl | 1 | H | |
| 26 | 4-F | Cl | 2 | H | 142-145 |
| 27 | 4-F | Cl | 3 | H | 143-146 |
| 28 | 4-F | Cl | 4 | H | |
| 29 | 4-Cl | Cl | 1 | H | |
| 30 | 4-Cl | Cl | 2 | H | |
| 31 | 4-Cl | Cl | 3 | H | |
| 32 | 4-Cl | Cl | 4 | H | |
| 33 | H | F | 1 | H | |
| 34 | H | F | 2 | H | |
| 35 | H | F | 3 | H | |
| 36 | H | F | 4 | H | |
| 37 | 4-F | F | 1 | H | |
| 38 | 4-F | F | 2 | H | |
| 39 | 4-F | F | 3 | H | |
| 40 | 4-F | F | 4 | H | |
| 41 | 4-Cl | F | 1 | H | |
| 42 | 4-Cl | F | 2 | H | |
| 43 | 4-Cl | F | 3 | H | |
| 44 | 4-Cl | F | 4 | H | |
| 45 | H | CF₃ | 1 | H | |
| 46 | H | CF₃ | 2 | H | |
| 47 | H | CF₃ | 3 | H | |
| 48 | H | CF₃ | 4 | H | |
| 49 | 4-F | CF₃ | 1 | H | |
| 50 | 4-F | CF₃ | 2 | H | |
| 51 | 4-F | CF₃ | 3 | H | |
| 52 | 4-F | CF₃ | 4 | H | |
| 53 | H | OCHF₂ | 1 | H | |
| 54 | H | OCHF₂ | 2 | H | |
| 55 | H | OCHF₂ | 3 | H | |
| 56 | H | OCHF₂ | 4 | H | |
| 57 | 4-F | OCHF₂ | 1 | H | |
| 58 | 4-F | OCHF₂ | 2 | H | |
| 59 | 4-F | OCHF₂ | 3 | H | 128-131 |
| 60 | 4-F | OCHF₂ | 4 | H | |
| 61 | 4-Cl | CF₃ | 1 | H | |
| 62 | 4-Cl | CF₃ | 2 | H | |
| 63 | 4-Cl | CF₃ | 3 | H | |
| 64 | 4-Cl | CF₃ | 4 | H | |
| 65 | 4-Cl | OCHF₂ | 1 | H | |
| 66 | 4-Cl | OCHF₂ | 2 | H | |
| 67 | 4-Cl | OCHF₂ | 3 | H | |
| 68 | 4-Cl | OCHF₂ | 4 | H | |
| 69 | 4-CHO | Methyl | 3 | H | 151-154 |
| 70 | 4-CN | Methyl | 3 | H | 178-181 |
| 71 | 4-CF₃ | Methyl | 3 | H | 189-192 |
| 72 | 4-F | Ethoxy | 2 | H | 135-138 |
| 73 | 4-F | Ethoxy | 1 | H | 114-117 |
| 74 | H | Ethoxy | 2 | H | 140-143 |
| 75 | H | Ethoxy | 3 | H | 102-105 |
| 76 | H | Ethoxy | 1 | H | 120-123 |
| 77 | 4-F | Ethoxy | 3 | H | 130-133 |
| 78 | 4-F | Methyl | 2 | 7,7-Dimethyl | 171-173 |
| 79 | 4-F | Methyl | 4 | H | 160-162 |
| 80 | 4-Cl | Ethoxy | 1 | H | 139-142 |
| 81 | 4-Cl | Ethoxy | 3 | H | 139-142 |
| 82 | 4-CH₃ | Methyl | 3 | H | 56-59 |
| 83 | 4-CHO | Methyl | 3 | H | 128-131 |
| 84 | 4-Methoxy | Methyl | 3 | H | 139-142 |
| 85 | 2,4-Difluor | Methyl | 3 | H | 131-134 |
| 86 | 2-Cl | Methyl | 3 | H | 120-123 |
| 87 | 2-F | Methyl | 3 | H | 123-126 |
| 88 | 4-CH(=NOCH₃) | Methyl | 3 | H | 143-146 |
| 89 | 4-tert-Butyl | Methyl | 3 | H | 178-181 |
| 90 | 4-Isopropyl | Methyl | 3 | H | 135-138 |
| 91 | 2-Methyl | Methyl | 3 | H | 2,8 (m); 3,1 (m); 7-2-7,3 (m); 7,7 (m) |
| 92 | 4-CN | Methyl | 3 | H | 141-144 |
| 93 | 4-C(=NOCH₃)(CH₃) | Methyl | 2 | H | 188-191 |
| 94 | 4-SCH₃ | Methyl | 2 | H | 160-163 |
| 95 | 3-SCH₃ | Methyl | 2 | H | 151-154 |
| 96 | 4-Cl | Methyl | 2 | 7,7-Dimethyl | 181-183 |
| 97 | 4-CF₃ | Methyl | 2 | 7,7-Dimethyl | 189-190 |
| 98 | 4-Methyl | Methyl | 2 | 7,7-Dimethyl | 185-186 |
| 99 | 4-Ethyl | Methyl | 2 | 7,7-Dimethyl | 164-166 |
| 100 | 4-Isopropyl | Methyl | 2 | 7,7-Dimethyl | 181-182 |
| 101 | 4-tert-Butyl | Methyl | 2 | 7,7-Dimethyl | 201 |
| 102 | 4-Methoxy | Methyl | 2 | 7,7-Dimethyl | 199-201 |
| 103 | 4-SCH₃ | Methyl | 2 | 7,7-Dimethyl | 187-188 |
| 104 | 4-OCF₃ | Methyl | 2 | 7,7-Dimethyl | 197-198 |
| 105 | 4-Ethoxy | Methyl | 2 | 7,7-Dimethyl | 1,0 (s); 3,0 (m); 4,1 (q); 6,9 (m); 8,2 (m) |
| 106 | 4-Propoxy | Methyl | 2 | 7,7-Dimethyl | 172-174 |
| 107 | 4-Isopropoxy | Methyl | 2 | 7,7-Dimethyl | 183-184 |
| 108 | 4-Phenoxy | Methyl | 2 | 7,7-Dimethyl | 153-155 |
| 109 | 4-Nitro | Methyl | 2 | 7,7-Dimethyl | 1,0 (s); 1,7 (m); 3,0 (m); 8,3 (m); 8,4 (m) |
| 110 | 4-CN | Methyl | 2 | 7,7-Dimethyl | 212-215 |
| 111 | 4-CHO | Methyl | 2 | 7,7-Dimethyl | 164-166 |
| 112 | 4-C(H)(=NOCH₃) | Methyl | 2 | 7,7-Dimethyl | 195-198 |
| 113 | 4-COCH₃ | Methyl | 2 | 7,7-Dimethyl | 197-199 |
| 114 | 4-C(=NOCH₃)(CH₃) | Methyl | 2 | 7,7-Dimethyl | 186-187 |
| 115 | 4-C(=NOCH₂H₃)CH₃ | Methyl | 2 | 7,7-Dimethyl | 174-175 |
| 116 | 4-CO₂CH₃ | Methyl | 2 | 7,7-Dimethyl | 1,0 (s); 3,95 (s); 3,0 (m); 7,7 (m); 8,35 (m) |
| 117 | 2-F | Methyl | 2 | 7,7-Dimethyl | 155 |
| 118 | 2-Cl | Methyl | 2 | 7,7-Dimethyl | 102-106 |
| 119 | 2-Methyl | Methyl | 2 | 7,7-Dimethyl | 126-128 |
| 120 | 2-Methoxy | Methyl | 2 | 7,7-Dimethyl | 128-131 |
| 121 | 2-Ethoxy | Methyl | 2 | 7,7-Dimethyl | 117-120 |
| 122 | 2,3-Difluor | Methyl | 2 | 7,7-Dimethyl | 140-143 |
| 123 | 2,3-Dimethyl | Methyl | 2 | 7,7-Dimethyl | 153-156 |
| 124 | 2,4-Difluor | Methyl | 2 | 7,7-Dimethyl | 162-166 |
| 125 | 2-Fluor-4-methyl | Methyl | 2 | 7,7-Dimethyl | 162-166 |
| 126 | 2-Methyl-4-fluor | Methyl | 2 | 7,7-Dimethyl | 148-152 |
| 127 | 2,4-Dimethoxy | Methyl | 2 | 7,7-Dimethyl | 134-137 |
| 128 | 2,5-Difluor | Methyl | 2 | 7,7-Dimethyl | 175-177 |
| 129 | 2,5-Dichlor | Methyl | 2 | 7,7-Dimethyl | 137-140 |
| 130 | 2,5-Dimethyl | Methyl | 2 | 7,7-Dimethyl | 104-110 |
| 131 | 3-Fluor | Methyl | 2 | 7,7-Dimethyl | 158-160 |
| 132 | 3-Chlor | Methyl | 2 | 7,7-Dimethyl | 174-176 |
| 133 | 3-Methyl | Methyl | 2 | 7,7-Dimethyl | 133-135 |
| 134 | 3-CF₃ | Methyl | 2 | 7,7-Dimethyl | 127-128 |
| 135 | 3-Methoxy | Methyl | 2 | 7,7-Dimethyl | 143-145 |
| 136 | 3-SCH₃ | Methyl | 2 | 7,7-Dimethyl | 119-121 |
| 137 | 3-Ethoxy | Methyl | 2 | 7,7-Dimethyl | 147-151 |
| 138 | 3-Isopropoxy | Methyl | 2 | 7,7-Dimethyl | 127-130 |
| 139 | 3-COCH₃ | Methyl | 2 | 7,7-Dimethyl | 151-152 |
| 140 | 3-C(=NOCH₃)CH₃ | Methyl | 2 | 7,7-Dimethyl | 146-148 |
| 141 | 3-C(=NOCH₂CH₃)CH₃ | Methyl | 2 | 7,7-Dimethyl | 135-140 |
| 142 | 3-CO₂CH₃ | Methyl | 2 | 7,7-Dimethyl | 132-135 |
| 143 | 3,4-Difluor | Methyl | 2 | 7,7-Dimethyl | 185-188 |
| 144 | 3,4-Dichlor | Methyl | 2 | 7,7-Dimethyl | 182-186 |
| 145 | 3,4-Dimethyl | Methyl | 2 | 7,7-Dimethyl | 153-156 |
| 146 | 3,4-Dimethoxy | Methyl | 2 | 7,7-Dimethyl | 171-175 |
| 147 | 3-Fluor-4-methyl | Methyl | 2 | 7,7-Dimethyl | 187-188 |
| 148 | 3-Fluor-4-methoxy | Methyl | 2 | 7,7-Dimethyl | 200-201 |
| 149 | 3-Fluor-4-ethoxy | Methyl | 2 | 7,7-Dimethyl | 179-181 |
| 150 | 3-Chlor-4-fluor | Methyl | 2 | 7,7-Dimethyl | 184 |
| 151 | 3-Chlor-4-ethoxy | Methyl | 2 | 7,7-Dimethyl | 118-120 |
| 152 | 3-Chlor-4-isopropoxy | Methyl | 2 | 7,7-Dimethyl | 133-137 |
| 153 | 3-Methyl-4-chlor | Methyl | 2 | 7,7-Dimethyl | 187 |
| 154 | 3-Methyl-4-methoxy | Methyl | 2 | 7,7-Dimethyl | 177-181 |
| 155 | 3,5-Difluor | Methyl | 2 | 7,7-Dimethyl | 1,0 (s); 1,7 (m); 2,4 (s); 2,5 (m); 3,0 (m) |
| 156 | 3,5-Dichlor | Methyl | 2 | 7,7-Dimethyl | 118-123 |
| 157 | 3,5-Dimethyl | Methyl | 2 | 7,7-Dimethyl | 115-120 |
| 158 | 3,5-(CF₃)₂ | Methyl | 2 | 7,7-Dimethyl | 149-151 |
| 159 | 3,4,5-Trifluor | Methyl | 2 | 7,7-Dimethyl | 194-195 |
| 160 | 4-COCH₃ | Methyl | 3 | H | 161-164 |
| 161 | 3,4-Dichlor | Methyl | 3 | H | 157-160 |
| 162 | 3-Cl | Methyl | 3 | H | 123-126 |
| 163 | 3-F | Methyl | 3 | H | 121-125 |
| 164 | 3-CH₃ | Methyl | 3 | H | 100-103 |
| 165 | 3-OCH₃ | Methyl | 3 | H | 115-118 |
| 166 | 3,4-Dimethoxy | Methyl | 3 | H | 148-151 |
| 167 | 3,5-Dichlor | Methyl | 3 | H | 2,5 (s); 2,8 (m); 3,1 (m); 8,4 (m); 8,6 (m) |
| 168 | 3-Cl-4-F | Methyl | 3 | H | 131-134 |
| 169 | 4-Phenoxy | Methyl | 3 | H | 161-164 |
| 170 | 3-Brom | Methyl | 3 | H | 1,6-1,8 (m); 1,85-2,0 (s); 2,8 m); 3,1 (m); |
| 171 | 4-SCH₃ | Methyl | 3 | H | 147-150 |
| 172 | 3-SCH₃ | Methyl | 3 | H | 115-118 |
| 173 | 3-CF₃ | Methyl | 3 | H | 95-98 |
| 174 | 3-Chlor-4-isopropoxy | Methyl | 3 | H | 99-102 |
| 175 | 3-Chlor-4-ethoxy | Methyl | 3 | H | 118-121 |
| 176 | 3,5-Dimethyl | Methyl | 3 | H | 2,8 (m); 3,1 (m); 7,7 (m); 8,3 (m); 8,5 (m) |
| 177 | 3-Fluor-4-methoxy | Methyl | 3 | H | 140-143 |
| 178 | 3,4-Difluor | Methyl | 3 | H | 155-158 |
| 179 | 2-Methoxy | Methyl | 3 | H | 173-176 |
| 180 | 4-Ethyl | Methyl | 3 | H | 151-154 |
| 181 | 3,4-Dimethyl | Methyl | 3 | H | 2,7 (m); 3,1 (m); 7,1 (m); 7,7 (m); 8,3 (m) |
| 182 | 2,4-Dimethoxy | Methyl | 3 | H | 2,2 (s); 3,7 (s); 3,8 (s); 6,5 (m); 6,6 (m) |
| 183 | 4-OCF₃ | Methyl | 3 | H | 156-159 |
| 184 | 2-Ethoxy | Methyl | 3 | H | 124-127 |
| 185 | 3,5-Difluor | Methyl | 3 | H | 170-173 |
| 186 | 3-Isopropoxy | Methyl | 3 | H | 1,3 (d); 2,4 (s); 4,6 (m); 6,9 (m); 7,1 (m) |
| 187 | 2,3-Difluor | Methyl | 3 | H | 132-135 |
| 188 | 2,5-Difluor | Methyl | 3 | H | 145-148 |
| 189 | 2,5-Dichlor | Methyl | 3 | H | 2,2 (s); 2,7 (m); 3,1 (m); 7,7 (m) |
| 190 | 2,3-Dimethyl | Methyl | 3 | H | 140-143 |
| 191 | 3-Methyl-4-fluor | Methyl | 3 | H | 120-123 |
| 192 | 3-CO₂CH₃ | Methyl | 3 | H | 2,2 (s); 2,8 (m); 3,1 (m); 3,9 (s) |
| 193 | 3,4,5-Trifluor | Methyl | 3 | H | 155-158 |
| 194 | 2-Methyl-4-fluor | Methyl | 3 | H | 2,1 (s); 2,2 (s); 2,7 (m); 3,1 (m); 8,3 (m); 8,5 (m) |
| 195 | 3-Methyl-4-methoxy | Methyl | 3 | H | 134-137 |
| 196 | 3-Methyl-4-chlor | Methyl | 3 | H | 134-137 |
| 197 | 3-Fluor-4-methyl | Methyl | 3 | H | 157-160 |
| 198 | 4-Isopropoxy | Methyl | 3 | H | 122-125 |
| 199 | 4-n-Butoxy | Methyl | 3 | H | 115-118 |
| 200 | 4-n-Propoxy | Methyl | 3 | H | 1,0 (m); 3,9 (m); 7,0 (m); 7,6 (m); 7,7 (m); 7,95 (m); 8,3 (m); 8,5 (m) |

| | | | | | |
|---|---|---|---|---|---|
| *Präfixe geben die jeweilige Position des (der) Substituenten an | | | | | |

### Prüfung der fungiziden Wirksamkeit:

Für die Anwendungbeispiele 1 bis 5 wurden die Wirkstoffe getrennt als eine Stammlösung aufbereitet mit 0,25 Gew.-% Wirkstoff in Aceton oder DMSO. Dieser Lösung wurde 1 Gew.-% Emulgator Wettol® EM 31 (Netzmittel mit Emulgier- und Dispergierwirkung auf der Basis ethoxylierter Alkylphenole) zugesetzt und entsprechend der gewünschten Konzentration mit Wasser verdünnt.

Für die Anwendungsbeispiele 6 bis 9 wurden die Wirkstoffe als eine Stammlösung aufbereitet mit 25 mg Wirkstoff, welcher mit einem Gemisch aus Aceton und/oder DMSO und dem Emulgator Uniperol® EL (Netzmittel mit Emulgier- und Dispergierwirkung auf der Basis ethoxylierter Alkylphenole) im Volumen-Verhältnis Lösungsmittel-Emulgator von 99 zu 1 ad 10 ml aufgefüllt wurde. Anschließend wurde ad 100 ml mit Wasser aufgefüllt. Diese Stammlösung wurde mit dem beschriebenen Lösungsmittel-Emulgator-Wasser Gemisch zu der unten angegeben Wirkstoffkonzentration verdünnt.

### Anwendungsbeispiel 1 - Wirksamkeit gegen die Dürrfleckenkrankheit verursacht durch Alternaria solani

Blätter von Tomatenpflanzen der Sorte "Goldene Prinzessin" wurden mit einer wässrigen Suspension in der unten angegebenen Wirkstoffkonzentration bis zur Tropfnässe besprüht. Am nächsten Tag wurden die behandelten Pflanzen mit einer Sporenaufschwemmung von *Alternaria solani,* in einer 2%igen wässrigen Biomalzlösung mit einer Dichte von 0,17 x 10⁶ Sporen/ml infiziert. Anschließend wurden die Versuchspflanzen in einer mit Wasserdampf gesättigten Kammer bei Temperaturen von 20 bis 22°C aufgestellt. Nach 5 Tagen hatte sich die Krankheit auf den unbehandelten, jedoch infizierten Pflanzen so stark entwickelt, dass der Befall visuell ermittelt werden konnte.

In diesem Test zeigten die mit 250 ppm der Wirkstoffe aus den Beispielen 1, 6, 7, 9 und 11 behandelten Pflanzen keinen Befall, während die unbehandelten Pflanzen zu 90 % befallen waren. Die zu Vergleichszwecken unter den gleichen Bedingungen mit 2-(5-Methyl-6-(4-fluorphenyl)pyridin-2-yl)-5,6,7,8-tetrahydrochinazolin (Verbindung gemäß EP-A 259 139) behandelten Pflanzen zeigten einen Befall von 80 %. Anwendungsbeispiel 2 - Wirksamkeit gegen den Grauschimmel an Paprikablättern, verursacht durch *Botrytis cinerea* bei protektiver Anwendung

Paprikablätter der Sorte "Neusiedler Ideal Elite" wurden, nachdem sich 2 bis 3 Blätter gut entwickelt hatten, mit einer wässrigen Suspension in der unten angegebenen Wirkstoffkonzentration bis zur Tropfnässe besprüht. Am nächsten Tag wurden die behandelten Pflanzen mit einer wässrigen Sporensuspension von *Botrytis cinerea* in einer 2%igen wässrigen Biomalzlösung mit einer Dichte von 1,7 x 10⁶ Sporen/ml inokuliert. Anschließend wurden die Pflanzen in einer Klimakammer bei Temperaturen zwischen 22 und 24°C und hoher Luftfeuchtigkeit aufgestellt. Nach 5 Tagen wurde das Ausmaß des Pilzbefalls visuell anhand des Befalls der Blattfläche ermittelt.

In diesem Test zeigten die mit 250 ppm der Wirkstoffe aus den Beispielen 6, 7 und 11 behandelten Pflanzen keinen Befall, während die unbehandelten Pflanzen zu 90 % befallen waren.

### Anwendungsbeispiel 3 - Wirksamkeit gegen Weizenmehltau, verursacht durch Erysiphe [syn. Blumerial graminis forma specialis. tritici

Blätter von in Töpfen gewachsenen Weizenkeimlingen der Sorte "Kanzler" wurden mit wässriger Suspension in der unten angegebenen Wirkstoffkonzentration bis zur Tropfnässe besprüht. 24 h nach dem Antrocknen des Spritzbelags wurden die Blätter mit den Sporen des Weizenmehltaus (*Erysiphe [syn. Blumeria] graminis forma specialis. tritici*) bestäubt. Anschließend wurden die Pflanzen im Gewächshaus bei Temperaturen zwischen 20 und 24°C und 60 - 90 % relativer Luftfeuchtigkeit aufgestellt. Nach 7 Tagen wurde das Ausmaß des Pilzbefalls visuell anhand des Befalls der Blattfläche ermittelt.

In diesem Test zeigten die mit 250 ppm des Wirkstoffs aus Beispiel 7 behandelten Pflanzen nur einen geringfügigen Befall (20 %), während die unbehandelten Pflanzen zu 90 % befallen waren. Die zu Vergleichszwecken unter den gleichen Bedingungen mit 2-(5-Methyl-6-(4-fluorphenyl)pyridin-2-yl)-5,6,7,8-tetrahydrochinazolin (Verbindung gemäß EP-A 259 139) behandelten Pflanzen zeigten einen Befall von 80 %.

### Anwendungsbeispiel 4 - Wirksamkeit gegen Rebenperonospora, verursacht durch Plasmopara viticola

Blätter von Topfreben wurden mit wässriger Suspension in der unten angegebenen Wirkstoffkonzentration bis zur Tropfnässe besprüht. Am nächsten Tag wurden die Unterseiten der Blätter mit einer wässrigen Sporangienaufschwemmung von *Plasmopara viticola* inokuliert. Danach wurden die Reben zunächst für 48 h in einer mit Wasserdampf gesättigten Kammer und anschließend im Gewächshaus bei Temperaturen zwischen 20 und 30 °C aufgestellt. Nach dieser Zeit wurden die Pflanzen zur Beschleunigung des Sporangienausbruchs abermals 16 h in die mit Wasserdampf gesättigte Kammer gestellt. Dann wurde das Ausmaß des Befallsentwicklung auf den Blattunterseiten ermittelt.

In diesem Test zeigten die mit 63 ppm des Wirkstoffs aus Beispiel 7 behandelten Pflanzen nur keinen Befall, während die unbehandelten Pflanzen zu 90 % befallen waren. Die zu Vergleichszwecken unter den gleichen Bedingungen mit 2-(5-Methyl-6-(4-fluorphenyl)pyridin-2-yl)-5,6,7,8-tetrahydrochinazolin (Verbindung gemäß EP-A 259 139) behandelten Pflanzen zeigten einen Befall von 70 %.

### Anwendungsbeispiel 5 - Kurative Wirksamkeit gegen Weizenbraunrost verursacht durch Puccinia recondita

Blätter von in Töpfen gewachsenen Weizensämlingen der Sorte "Kanzler" wurden mit einer Sporensuspension des Braunrostes (*Puccinia recondita*) inokuliert. Danach wurden die Töpfe für 24 Stunden in eine Kammer mit hoher Luftfeuchtigkeit (90 bis 95 %) und 20 bis 22 °C gestellt. Während dieser Zeit keimten die Sporen aus und die Keimschläuche drangen in das Blattgewebe ein. Die infizierten Pflanzen wurden am nächsten Tag mit einer wässriger Suspension in der unten angegebenen Wirkstoffkonzentration bis zur Tropfnässe besprüht. Die Suspension oder Emulsion wurde wie oben beschrieben hergestellt. Nach dem Antrocknen des Spritzbelages wurden die Versuchspflanzen im Gewächshaus bei Temperaturen zwischen 20 und 22 ° C und 65 bis 70 % relativer Luftfeuchte für 7 Tage kultiviert. Dann wurde das Ausmaß der Rostpilzentwicklung auf den Blättern ermittelt.

In diesem Test zeigten die mit 250 ppm des Wirkstoffs aus Beispiel 9 behandelten Pflanzen nur einen geringfügigen Befall (20 % Befall), während die unbehandelten Pflanzen zu 90 % befallen waren. Die zu Vergleichszwecken unter den gleichen Bedingungen mit 2-(5-Methyl-6-(4-fluorphenyl)pyridin-2-yl)-5,6,7,8-tetrahydrochinazolin (Verbindung gemäß EP-A 259 139) behandelten Pflanzen zeigten einen Befall von 50 %.

### Anwendungsbeispiel 6 - Wirksamkeit gegen die Dürrfleckenkrankheit verursacht durch Alternaria solani

Blätter von getopften Tomatenpflanzen der Sorte "Goldene Königin" wurden mit einer wässrigen Suspension in der unten angegebenen Wirkstoffkonzentration bis zur Tropfnässe besprüht. Am nächsten Tag wurden die Blätter mit einer wässrigen Sporenaufschwemmung von *Alternaria solani* in einer 2%igen Biomalzlösung mit einer Dichte von 0,17 x 10⁶ Sporen/ml infiziert. Anschließend wurden die Pflanzen in einer mit Wasserdampf gesättigten Kammer bei Temperaturen von 20 bis 22°C aufgestellt. Nach 5 Tagen hatte sich die Krankheit auf den unbehandelten, jedoch infizierten Pflanzen so stark entwickelt, dass der Befall visuell ermittelt werden konnte.

In diesem Test zeigten die mit 63 ppm der Wirkstoffe aus den Beispielen 9, 13, 16, 17, 18, 72, 73, 74, 75, 76, 78, 79, 82, 84, 85, 86, 87, 88, 89, 90, 91, 92, 93 und 95 behandelten Pflanzen maximal einen Befall von 20 %, während die unbehandelten Pflanzen zu 90 % befallen waren.

### Anwendungsbeispiel 7 - Wirksamkeit gegen die Netzfleckenkrankheit der Gerste verursacht durch Pyrenophora teres bei 1 Tag protektiver Anwendung

Blätter von in Töpfen gewachsenen Gerstenkeimlingen wurden mit wässriger Suspension in der unten angegebenen Wirkstoffkonzentration bis zur Tropfnässe besprüht. 24 Stunden nach dem Antrocknen des Spritzbelages wurden die Pflanzen mit einer wässrigen Sporensuspension von *Pyrenophora [syn. Drechslera] teres,* dem Erreger der Netzfleckenkrankheit, inokuliert. Anschließend wurden die Versuchspflanzen im Gewächshaus bei Temperaturen zwischen 20 und 24°C und 95 bis 100 % relativer Luftfeuchtigkeit aufgestellt. Nach 6 Tagen wurde das Ausmaß der Krankheitsentwicklung visuell anhand des Befalls der Blattfläche in % ermittelt.

In diesem Test zeigten die mit 63 ppm der Wirkstoffe aus Beispielen 4, 9, 13, 16, 17, 18, 21, 23, 26, 27, 72, 73, 74, 75, 76, 77, 78, 79, 80, 81, 82, 83, 84, 85, 86, 87, 88, 89, 90, 91, 92, 93, 94, 95, 96, 98, 102, 105, 119, 120, 121, 122, 124, 126, 127, 128, 136, 137, 138, 139, 143, 146, 151, 157 und 159 behandelten Pflanzen maximal einen Befall von 20 %, während die unbehandelten Pflanzen zu 90 % befallen waren.

### Anwendungsbeispiel 8 - Wirksamkeit gegen den Grauschimmel an Paprikablättern, verursacht durch Botrytis cinerea bei protektiver Anwendung

Paprikablätter der Sorte "Neusiedler Ideal Elite" wurden, nachdem sich 2 bis 3 Blätter gut entwickelt hatten, mit einer wässrigen Suspension in der unten angegebenen Wirkstoffkonzentration bis zur Tropfnässe besprüht. Am nächsten Tag wurden die behandelten Pflanzen mit einer Sporensuspension von *Botrytis cinerea,* die 1,7 x 10⁶ Sporen/ml in einer 2%igen wässrigen Biomalzlösung enthielt, inokuliert. Anschließend wurden die Versuchspflanzen in einer Klimakammer bei Temperaturen zwischen 22 und 24 °C, Dunkelheit und hoher Luftfeuchtigkeit aufgestellt. Nach 5 Tagen wurde das Ausmaß des Pilzbefalls visuell in % anhand des Befalls der Blattfläche ermittelt.

In diesem Test zeigten die mit 63 ppm der Wirkstoffe aus den Beispielen 4, 26, 80, 83 und 94 behandelten Pflanzen maximal einen Befall von 20 %, während die unbehandelten Pflanzen zu 90 % befallen waren.

### Anwendungsbeispiel 9 - Aktivität gegen die Krautfäule an Tomaten verursacht durch Phytophthora infestans bei protektiver Behandlung

Blätter von getopften Tomatenpflanzen wurden mit einer wässriger Suspension in der unten angegebenen Wirkstoffkonzentration bis zur Tropfnässe besprüht. Am folgenden Tag wurden die Blätter mit einer wässrigen Sporangienaufschwemmung von *Phytophthora infestans* infiziert. Anschließend wurden die Pflanzen in einer wasserdampfgesättigten Kammer bei Temperaturen zwischen 18 und 20°C aufgestellt. Nach 6 Tagen hatte sich die Krautfäule auf den unbehandelten, jedoch infizierten Kontrollpflanzen so stark entwickelt, dass der Befall visuell in % ermittelt werden konnte.

In diesem Test zeigten die mit 63 ppm der Wirkstoffe aus Beispielen 98, 117, 118, 119, 120, 122, 124, 125, 126, 127, 128, 129, 130, 133, 134, 135, 136, 138, 139, 141, 142, 143, 144, 145, 146, 147, 150, 151, 152, 156, 157 und 159 behandelten Pflanzen maximal einen Befall von 20 %, während die unbehandelten Pflanzen zu 90 % befallen waren.

## Patentansprüche

1. 2-(Pyridin-2-yl)-pyrimidin-Verbindungen der allgemeinen Formel I 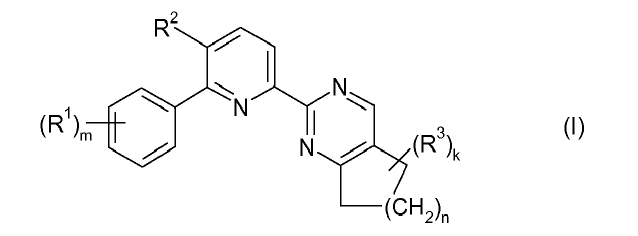 worin:
k für 0, 1, 2 oder 3 steht;
m für 0, 1, 2, 3, 4 oder 5 steht;
n für 3, 4 oder 5 steht;
R¹ unabhängig voneinander Halogen, OH, CN, NO₂, C₁-C₄-Alkyl, C₁-C₄-Halogenalkyl, C₁-C₄-Alkoxy, C₁-C₄-Halogenalkoxy, C₂-C₄-Alkenyl, C₂-C₄-Alkinyl, C₃-C₈-Cycloalkyl, C₁-C₄-Alkoxy-C₁-C₄-alkyl, Amino, Phenoxy, das gegebenenfalls durch Halogen oder C₁-C₄-Alkyl substituiert ist, NHR, NR₂, C(R^{a})=N-OR^{b}, S(=O)ₚA¹ oder C(=O)A² bedeuten, wobei p, R, R^{a}, R^{b}, A¹ und A² die folgenden Bedeutungen haben:
R C₁-C₄-Alkyl oder C₁-C₄-Alkylcarbonyl,
R ^{a} Wasserstoff oder C₁-C₄-Alkyl;
R ^{b} C₁-C₄-Alkyl, C₃-C₄-Alkenyl oder C₃-C₄-Alkinyl,
p 0, 1 oder 2,
A¹ C₁-C₄-Alkyl, oder für p = 2 auch NH₂, C₁-C₄-Alkylamino oder Di-(C₁-C₄-alkyl)amino, und
A² Wasserstoff, Hydroxy, C₁-C₄-Alkyl, C₁-C₄-Alkylamino, Di-(C₁-C₄-alkyl)amino, C₂-C₄-Alkenyl, C₁-C₄-Alkoxy oder C₁-C₄-Halogenalkoxy; oder zwei an benachbarte C-Atome gebundene Reste R¹ auch gemeinsam für eine Gruppe -O-Alk-O- stehen können, worin Alk für lineares oder verzweigtes C₁-C₄-Alkylen steht, worin 1, 2, 3 oder 4 Wasserstoffatome auch durch Halogen ersetzt sein können;
R² Wasserstoff oder C₁-C₄-Alkyl bedeutet;
R³ für C₁-C₄-Alkyl steht;
und die landwirtschaftlich verträglichen Salze der Verbindungen I.

2. Verbindungen der Formel I gemäß Anspruch 1, worin n für 3 steht.

3. Verbindungen der Formel I gemäß einem der vorhergehenden Ansprüche, worin m für 0, 1, 2 oder 3 steht.

4. Verbindungen der Formel I gemäß einem der vorhergehenden Ansprüche, worin k für 0 steht.

5. Verbindungen der Formel I gemäß einem der vorhergehenden Ansprüche, worin R¹ für Halogen, CN, C₁-C₄-Alkyl, C₁-C₄-Halogenalkyl, C₁-C₄-Alkoxy oder C₁-C₄-Halogenalkoxy steht.

6. Verbindungen der Formel I gemäß einem der Ansprüche 1 bis 3, worin einer der Reste R¹ für eine Gruppe C(R^{a})=N-OR^{b} steht.

7. Verbindungen der Formel I gemäß einem der vorhergehenden Ansprüche, worin die Gruppe 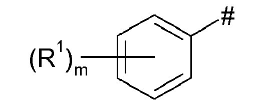 worin # die Verknüpfungsstelle mit dem Pyridinring ist, für einen Rest der Formel P 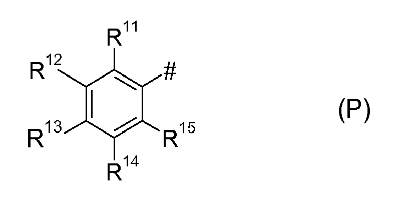 steht, worin
R¹¹ Wasserstoff, Fluor, Chlor, CH₃, OCH₃, OCHF₂, OCF₃ oder CF₃ bedeutet;
R¹², R¹⁴ unabhängig voneinander Wasserstoff, Chlor, Fluor, CH₃, OCH₃, OCHF₂, OCF₃ oder CF₃ bedeuten, wobei einer der Reste R¹² und R¹⁴ auch für NO₂, C(O)CH₃ oder COOCH₃ stehen kann;
R¹³ Wasserstoff, Fluor, Chlor, Cyano, OH, CHO, NO₂, NH₂, Methylamino, Dimethylamino, Diethylamino, C₁-C₄-Alkyl, C₃-C₈-Cycloalkyl, C₁-C₄-Alkoxy, C₁-C₄-Alkylthio, C₁-C₄-Haloalkyl, C₁-C₄-Haloalkoxy, CO(A²), worin A² für C₁-C₄-Alkyl oder C₁-C₄-Alkoxy steht, oder eine Gruppe C(R^{a})=NOR^{b}, worin R^{a} für Wasserstoff oder Methyl steht und R^{b} für C₁-C₄-Alkyl, Propargyl oder Allyl steht, bedeutet oder R¹² und R¹³ gemeinsam eine Gruppe O-CH₂-O bilden; und
R¹⁵ Wasserstoff, Fluor, Chlor oder C₁-C₄-Alkyl bedeutet.

8. Verbindungen der Formel I nach Anspruch 7, worin wenigstens einer der Reste R¹¹ R¹² R¹³ R¹⁴ oder R¹⁵ von Wasserstoff verschieden ist.

9. Verwendung einer Verbindung der allgemeinen Formel I gemäß einem der vorhergehenden Ansprüche sowie der landwirtschaftlich verträglichen Salze von I zur Bekämpfung von pflanzenpathogenen Pilzen.

10. Zur Bekämpfung von Schadpilzen geeignetes Mittel, enthaltend einen festen oder flüssigen Trägerstoff und eine Verbindung der Formel I gemäß einem der Ansprüche 1 bis 9 und/oder ein landwirtschaftlich verträgliches Salz von I.

11. Verfahren zur Bekämpfung von pflanzenpathogenen Pilzen, **dadurch gekennzeichnet, dass** man die Pilze oder die vor Pilzbefall zu schützenden Materialien, Pflanzen, den Boden oder Saatgüter mit einer wirksamen Menge einer Verbindung der Formel I gemäß einem der Ansprüche 1 bis 9 behandelt und/oder eines landwirtschaftlich verträglichen Salzes von I behandelt.
